# EUROPEAN PATENT APPLICATION

(11) **EP 3 100 740 A1**
(43) Date of publication of application: **07.12.2016**
(21) Application number: 14880740.7
(22) Date of filing: 30.07.2014
(51) Int. Cl.: A61K 38/16, A61K 38/10, A01N 1/02

(54) **COMPOSITION AND METHOD FOR EX VIVO IMMUNOMODULATION AND/OR PRESERVATION OF ORGANS, METHODS AND USE**

(30) Priority: 30.01.2014 BR 1402362
(71) Applicant: União Brasileira De Educação E Assistência - Mantenedora Da Pucrs, CEP: 90619-900 Porto Alegre - RS (BR)
(72) Inventor: BONORINO, Cristina Beatriz Cazabuena, CEP 91410-330 Porto Alegre - RS (BR); BORGES, Thiago de Jesus, 90035-120 Porto Alegre - RS (BR)
(74) Representative: Trillat, Anne-Cecile
(86) International application number: PCT/BR2014/000258
(87) International publication number: WO 2015/113126

(57) **Abstract**

The present invention provides a composition for modulating MARCH-I, MHC II and/or CD86 in the cells of an organ *ex vivo,* that is to say an organ taken from a donor for subsequent transplantation in a recipient. In one embodiment, the said composition comprises one or more organ preservation solutions in combination with an Hsp. In another embodiment, the composition of the invention comprises Hsp70 and/or one or more synthetic Hsp70-derived peptides. The composition of the invention provides preservation while at the same time reducing rejection of an organ, thus offering many advantages and solving various relevant technical problems: not only preserving the useful life of the organ but also initiating a process in the organ which inhibits acute rejection; lengthening the useful life of the organ *in vivo;* having local immunomodulating effects in the recipient; having an indirect impact on the quality of life of the transplant recipient by necessitating fewer immunosuppressive medications; inducing the activity of regulatory T cells (which are acknowledged to be immunosuppressors), reducing the expression of MHC molecules in the graft, reducing alloreactive expression in the recipient's lymph nodes, and lengthening the survival of the graft in the recipient.

SEQUENCE LISTING

<110>  União Brasileira de Educação e Assistência - Mantenedora da
        PUCRS

<120>  COMPOSITION AND METHOD FOR EX-VIVO IMMUNOMODULATION AND/OR
       PRESERVATION OF ORGANS, PROCESS AND USE

<130>  Solução Preservação (A0001-12)

<160>  7

<170>  PatentIn version 3.5

<210>  1
<211>  15
<212>  PRT
<213>  Artificial Sequence

<220>
<223>  Heat Shock Protein HSP70

<400>  1

Val Gly Gly Ser Thr Arg Met Pro Ala Val Thr Asp Leu Val Lys
1               5                   10                  15


<210>  2
<211>  16
<212>  PRT
<213>  Artificial Sequence

<220>
<223>  Heat Shock Protein HSP70

<400>  2

Asp Lys Met Ala Met Gln Arg Leu Arg Glu Ala Ala Glu Lys Ala Lys
1               5                   10                  15


<210>  3
<211>  15
<212>  PRT
<213>  Artificial Sequence

<220>
<223>  Heat Shock Protein HSP70

<400>  3

Lys Gly Gly Val Met Thr Arg Leu Ile Glu Arg Asn Thr Thr Ile
1               5                   10                  15


<210>  4
<211>  15
<212>  PRT
<213>  Artificial Sequence

<220>
<223>  Heat Shock Protein HSP70

<400>  4

Pro Arg Gly Ile Pro Gln Ile Glu Val Thr Phe Asp Ile Asp Ala
1               5                   10                  15


<210>  5
<211>  15
<212>  PRT
<213>  Artificial Sequence

<220>
<223>  Heat Shock Protein HSP70

<400>  5

Lys Tyr Thr Ala Pro Glu Ile Ser Ala Arg Ile Leu Met Lys Leu
1               5                   10                  15


<210>  6
<211>  15
<212>  PRT
<213>  Artificial Sequence

<220>
<223>  Heat Shock Protein HSP70

<400>  6

Ile Thr Asp Ala Val Ile Thr Thr Pro Ala Tyr Phe Asn Asp Ala
1               5                   10                  15


<210>  7
<211>  15
<212>  PRT
<213>  Artificial Sequence

<220>
<223>  Heat Shock Protein HSP70

<400>  7

Gly Ser Asp Ile Ser Ala Ile Lys Ser Ala Met Glu Lys Leu Gly
1               5                   10                  15

## Description

### Related Patent Application

The present patent application claims the Brazilian patent application priority BR 10 2014 002362 3, filed on January 30, 2014.

### Field of the Invention

The invention relates to the fields of Immunology, Pharmacy, Chemistry and Medicine. More specifically, the present invention provides a composition for *ex-vivo* modulating MARCH-I, MHC II and/or CD86 in organ cells, i.e., removed from the donor to subsequent transplantation to a recipient. In an embodiment, the said composition comprises one or more organs preserving solutions together with an Hsp. In another embodiment, the composition of the invention comprises the Hsp70 and/or one or more synthetic peptides derived from Hsp70. The composition of the invention provides the preservation and at the same time rejection reduction of the organ, then providing numerous advantages and solving several relevant technical problems: not only preserves the useful life of the organ as well as starting a process in the organ that inhibits the acute rejection; lengths the *in vivo* useful life time of the organ; has local immunomodulating effects in the recipient; generates indirectly an impact in the life quality of the recipient when requiring less immunosuppressive medications; induces the regulatory T cells activity (which are admittedly immunosuppressive), reduces the MHC molecules expression in the graft, reduces the alloreactive response in lymph nodes of the recipient, lengths the graft survival in the recipient. Here, it is also disclosed a screening process of MARCH-I, MHC II and/or CD86 modulators for using in organs' preservation compositions organs and a process for preparing such compositions.

### Background of the Invention

An organ transplantation is successful depending on the immunosuppressive therapies which manage the organ's survival and also techniques preserving the donor organ appropriately. Regardless the source of the organ, all organs which are transplanted need to be preserved during the time of removal and implementation.

Currently, most part of the centers use the static storage in cold environment (*static cold preservation -* CS) for organs preservation. This technique of organs preservation is based on the fact that the metabolic rate of eukaryotic cells decline from two to three times at 10°C of reduction in the temperature in which they are. The said technique requires the blood fast removal, fast organ cooling and a balance between the preservation solution and the organ. But, the preservation conditions are extremely stressful and damaging to living tissues, as they may cause damages resulting from ischemia (preservation hypothermic conditions) and reperfusion (transplantation in the donor). The preservation technique in hypothermic conditions has been applied first in 1952 by Lefevbre and Nizet, in France. Since then, only a few advances have been achieved in the organs preservation.

Currently, the most used solution is the *University of Wisconsin (UW)* solution, which has been used since 1987. But there are other solutions used, such as the Histidine-Tryptophan-Ketoglutarate (HTK) solution, the Celsior solution, the Euro-Collins solution, among others. The most used preservation solutions currently preserve the visceral organs for up to 48 hours on average, depending on the organ condition. But they don't have the immunomodulating activities.

In addition to the appropriate preservation, people who deal with transplantation face another boarder area: the immune system of the recipient. The grafts rejection is a result of a complex and coordinated series of interactions involving the innate and adaptive immune system. The molecular basis of the rejection is in the T cells ability of recognizing polymorphic versions of proteins range, in such case, alloantigen resulting from a genetically different organism of the recipient. When the specific T cells recognize this alloantigen, they will proliferate, differentiate in effector cells and there will be a migration to the graft local where they are going to cause the tissue destruction (rejection). On the first day after the transplantation, the recipient monocytes start to enter into the transplanted tissue and dendritic cells (DCs) of the skin migrate, through lymphatic vessels, and infiltrate the draining lymph node of the recipient. In the draining lymph node, the DCs of the skin have antigens from the donor through two mechanisms: (a) direct way, in which the T cells recognize intact antigens of the MHC in the donor DCs; (b) indirect way, which involves the acknowledgment by the peptides T cells of the donor being presented in MHC molecules on the recipient's DCs surfaces. After some days, it is noted a large infiltrate of monocytes in the skin tissue, and the effort T cells (CD4+ and CD8+) start migrating laterally from the adjacent tissue to the graft. Finally, CD8+ T cells (cytotoxic) destroy the donor foreign cells, leading to necrosis and to final stages of rejection.

MARCH-I is a protein associated to the membrane and binds ubiquitin molecules to MHC II and CD86 molecules. This ubiquitination of the MHC II and CD86 molecules leads to its degradation and subsequent reduction of quantity/title in the membrane of antigens presenting cells, such as the dendritic cells (DCs). When the DCs are active, they reduce the MARCH-I expression and the levels of MHC II and CD86 increase in the membrane. On the other hand, when these cells are induced, the opposite occurs. The acute rejection starts when the active DCs found in the transplanted organ migrate to the draining lymph node of the recipient, interact with the T cells and activate them. This activation requires the presence of MHC II e CD86 molecules expressed by the DCs. Once active, these T cells come back to the graft, recognize it as something "strange" and start degrading it by inducting the cell death, causing the rejection of the organ. Historically, the organs rejection is treated in the clinic with drugs inhibiting the activation and the proliferation of the T cells. But a serious technical problem is that, by the lack of alternatives so far, the treatment has been made **in the patient and never in the donor organ,** which causes a systemic response and unwanted side effects, among other disadvantages. The present invention provides an innovative solution for these problems.

The immunosuppressive drugs currently used for the therapeutic treatment and handling of the organs rejection are focused on the inhibition of the alloreactive cell activation. However, they have several problems related to the induction of severe side effects. Among the severe side effects, we may point out: hypertension, which may lead to the situation where the patient needs additional medication for its control; nephrotoxicity, which occurs with up to 40% of the patients and frequently forces the doctor to deliver suboptimal doses of the drug for limiting the toxicity; effects over the central nervous system, such as: shivering, headache, depression, paresthesia, blurry vision, increased risk of viral, bacterial or fungal infections, increased risk of tumors occurrence, lack of appetite, nausea; some patients are resistant to the drugs and the combination of several drugs is necessary; high cost of the drugs; some drugs demonstrate adverse interactions with other drugs, such as antibiotics, non-steroidal anti-inflammatory, antiepileptic, antifungal and also immunization, such as German measles and polio.

Therefore, there is an enormous demand for developing new drugs or interventions which present lower side effects than the drugs available, or which reduce the necessity of their use. Also, new interventions may serve as alternative therapy for patients that are resistant to treatments used. The present invention solves this and another problems, providing a high potential at handling the rejection to organs.

Thermal shock or stress (Hsp) proteins were first described in 1962 and they form a group of proteins induced by stress signals, including environmental stresses (such as heat, for example) and pathophysiological states, such as fever, inflammation and infection, being distributed at an ubiquitous way between prokaryotic and eukaryotic organisms. The Hsps may be classified in five main families, according to their molecular weight: Hsp100, Hsp90, Hsp70, Hsp60 and sHsp (*small heat shock proteins*) and may be found in cytosol, membrane, core, endoplasmic reticulum and the mitochondria of the cells. These proteins work mainly as molecular chaperones, transporting proteins between cellular compartments, helping the folding of proteins being formed or at refolding the proteins that experienced damages, protecting thee aggregation of other proteins, apart from orienting proteins to degradation ways and helping the dissolution of protein complexes. In physiological conditions, the intracellular Hsp are distributed ubiquitously and maintain the function integrity of other cell proteins when exposed to a stress stimulus. This protecting function made that several research groups tried to induce the Hsp increase as a tentative of protecting the organ to be transplanted against ischemia damage and reperfusion. However, this data was not very promising.

Despite Hsps are considered intracellular proteins, they can be moved to the plasmatic membrane or released into the extracellular environment, interacting with cells from the immune system and changing its functions. The analysis of responses immune to Hsps in experimental models and patients has indicated the Hsp's abilities of promoting immunomodulation. Hps70 family members showed having a protector and anti-inflammatory role in animal models of arthritis, colitis, pulmonary fibrosis and brain damages. But how would Hsp70 be modulating the immune response? The first line of thought focuses on cells modulation of the innate immune system in an immunosuppressant profile. These studies showed this profile in dendritic cells, *myeloid-derived suppressor cells -* MDSCs, monocytes and macrophages. The other line of thought focuses on modulation of the adaptive immune system by Hsp70. The anti-inflammatory mechanism proposed is the induction of regulating T cells (Tregs) specific to peptides of Hsp70. The Tregs have a fundamental role in suppressing the effector immune responses that are excessive and that can cause damage to the tissue. These cells have an ability of inhibiting the proliferation of effector T cells (CD4+ and CD8+) in draining lymph nodes and they are also responsible for controlling its homeostasis. The Tregs can be used to control responses against non-proper antigens that are introduced in the host, as in the case of transplantations, and promote the indefinite survival of allografts in many experimental models.

The limitations in establishing immunosuppressant strategies in organ donation have led the present inventors to develop new approaches in the *ex-vivo* treatment of organs as new methods for the homeostatic mechanisms that prevent and limit inflammatory responses in the grafted tissues.

The research in the scientific and patent reading has pointed some documents partially relevant to the present invention which will be described in the following.

Document WO 2001/017554 describes methods and compositions for the treatment and rejection using proteins of thermal shock *(Heat shock proteins -* Hsp) to be administered to the patient. The said document describes compositions that can be used before, during and after the organ transplantation, including diminishing its rejection, being the said compositions preferably in the form of pharmaceutical compositions. The present invention distinguishes from the said document, among other technical reasons, for referring to peptides and method *for ex-vivo* immunomodulation and/or preservation of organs that comprises the application of at least one fragment of Hsp70 protein and/or an Hsp in combination with one or more organ preservation solutions.

Document US 5.348.945 refers to a method for fighting tissue mortality under stress and which stress induces to a response of endogenous Hsp70. The present invention differs from the said document, for it is not conjugated no *antigen* to HSP when treating immune cells presenting antigen for then treating recipient with the treated cells, modulating the rejection. In addition, it differs for the fact that immune cells presenting antigen are not treated aiming a subsequent cell therapy or concurrently to the transplantation. In the present invention the immune cells are treated *ex-vivo,* but inside the organ to be transplanted itself.

Document US 2006/0276389 A1 refers to a *heat shock protein* (HSP) or a *heat shock protein-like protein* (HSPLP) and that act as a fugetatic agent or chemiotactic negative. The present invention differs from the said document, among other technical reasons, for not referring to the HSP as a fugetatic agent or chemiotactic negative, also not having experimental evidences for such properties.

Document WO 2008/47243 A2 discloses a strategy of merging a Hsp70 with the transduction domain of another protein, in a way to transmit a death inhibitor signal to a cell. The present invention differs from such document, among other technical reasons, for not providing protein fusions. In addition, it does not refer to the cellular apoptosis problem itself but to the acute rejection *ex-vivo* problem to organ transplantations.

Document US 2002/0111314 A1 refers to a method for increasing the *heat shock protein* level in a cell. The present invention differs from the said document, among other technical reasons, for not referring to the intracellular increase of Hsp70, but to the extracellular treatment with Hsp70, as well as all other cells that compose the organ to be transplanted.

Document EP 2040539 B1 refers to compositions with nutrients and cofactors that lengthen the cell organ's lives, as well as corticoids for immunosuppression of the acute rejection. The present invention differs from the said document, among other technical reasons, for not being a typical organ preserving solution found in the state of the art. In addition, the composition of the present invention does not comprise corticoids.

The article *"Prolonged Survival of Allografts Induced by Mycobacterial Hsp70 ls Dependent on CD4+CD25+ Regulatory T Cells",* having the inventors as co-authors, disclose the immunomodulatory role of the Hsp70 in transplantation models, in a connected form to regulatory T cells. The study refers to an investigation of the Hsp70 ability of inhibiting the allograft system rejection in experimental models (a tumor model and another of non-vascularized tissue), in which treatment with has Hsp70 significantly lengthened briefly the graft survival or the tumor cells. In the article, evidences are presented of an immunosuppressant role of the Hsp70 protein in the rejection of only one non-vascularized tissue and tumor cells. However, nothing is quoted or even suggested about reducing the recipient's alloreactivity. In addition, despite the said article suggests the usage of Hsp70 in organ transplantation management, at any moment a composition for *ex-vivo* modulating MARCH-I, MHC II and/or CD86 in the cells of an organ is disclosed or suggested. Nor tests performed with real preservation compositions are performed.

Thus, none of the pointed documents describe a method for *ex-vivo* immunomodulation method and/or preservation of organs comprising the application of an effective dose of at least one of the molecular entities disclosed in the present invention, nor a composition for *ex-vivo* immunomodulation and preservation of transplantation organs comprising one or more organ preservation solutions (as, for example, a solution of Winscosin (Viaspan^{®}) or Euro-Collins (RENOGRAF^{®}), among others) in combination with one or more modelling molecule(s) of MARCH-I, MHC II and/or CD86 in organ cells already removed from donor, i.e., *ex-vivo.* Thus, the present invention provides immune system cells in organs that need *ex-vivo* conservation, in a way that they remain even induced, diminishing the all specific effector response.

According to what is showed from the researched literature, documents anticipating or suggesting the teachings of the present invention were not found, in a way that the solution here purposed, to the eyes of the inventors, has a new and inventive activity facing the state of the art.

### Summary of the Invention

The present invention solves the problems found in the state of the art related to the difficulty of maintaining the transplantation organs viability and the difficulty of limiting its acute rejection after the transplantation when reducing or excluding the use of immunosuppressant in the transplant recipient patient. The common inventive concept to different objects of the invention consists of modulating the strategy MARCH-I, MHC II and/or CD86 organ cells already removed from the donor, i.e., *ex-vivo,* instead of modulating the cells of the organ recipient person. The results achieved in the present invention show that this strategy lengths the useful life of a *in vivo* transplanted organ, in function of the acute rejection inhibitory effect provided by the MARCH-I, MHC II and/or CD86 modulation - clearly differs from the background known and with substantial technical benefits therefore not achieved. This inventive concept is implemented in several ways, all of them covered in the invention scope.

It is one of the invention objects the use of a substance modulating MARCH-I, MHC II and/or CD86 for preparing a composition for *ex-vivo* preservation and immunomodulation of organ cells to be transplanted.

It is another object of the invention a preservation composition of organs comprising:
- one or more organ preservation solutions; and
- a substance modulating MARCH-I, MHC II and/or CD86 in the organ cells to be transplanted, i.e., from the donor and already in the *ex-vivo* condition.

In one embodiment, said organ preservation solutions are chosen from the group consisting of: solution of Winscosin (Viaspan®); Euro-Collins (RENOGRAF^{®}); Histidine-Tryptophan-Ketoglutarate (HTK) solution - Custodiol^{®}; Celsior^{®} Cold Storage Solution; or combination thereof.

In one embodiment, the substance or molecular entity modulating MARCH-I, MHC II and/or CD86 is an Hsp (*heat shock protein*) selected from the group consisting of: Hsp70; Hsp60; Hsp40; Hsp90; Hsp110; fragment thereof or combinations of any of those molecular entities.

In one embodiment, the Hsp70 (DnaK) is the recombinant protein of *M*. *tuberculosis.* In one embodiment, the fragment of the protein Hsp70 is at least one peptide selected from the group consisting of peptides identified as: SEQ ID No:01, SEQ ID No:02, SEQ ID No:03, SEQ ID No:04, SEQ ID No:05, SEQ ID No:06 and SEQ ID No:07.

The present invention also provides a process for preparing an *ex vivo* immunomodulating composition for transplantation organ cells, said process comprising a step of mixing one or more organ preservation solutions with at least one modulating molecular entity of the amount of MARCH-I, MHC II and/or CD86.

It is another of the objects of the invention a method of *ex-vivo* immunomodulation of transplantation organs, said method comprising a step of *ex-vivo* contacting the organ cells with one or more substance(s) modulating MARCH-I, MHC II and/or CD86.

It is another of the objects of the invention a process for screening the new molecular entities useful for preparing the composition for *ex-vivo* preservation and/or immunomodulation of transplantation organs, said process being comprised of:
- a first step of contacting one or more molecular entity(ies) with cells having a protein selected from the group consisting of MARCH-I, MHC II, CD86, or combinations thereof;
- a second step of evaluating the modulation of the amount of MARCH-I, MHC II and/or CD86 in the cells treated in this way; and
- a third step of selecting the molecular entities with major potential of modulation of the amount of MARCH-I, MHC II and/or CD86 in the cells treated in this way.

In one embodiment, said process is characterized by the fact that the said cells having a protein selected from the group consisting of MARCH-I, MHC II, CD86, or combination thereof, are dendritic cells or cells of the organ to be transplanted itself.

In one embodiment, the said process is characterized by the fact that the step of evaluating the MARCH-I, MHC II and/or CD86 amount in the cells is done by measuring through PCR in real time the mRNA transcribed from genes codifying MARCH-I, MHC II and/or CD86.

In one embodiment, said process further comprises the step of *in vivo* evaluation of the cell viability and/or the presence of cell damage in the *ex-vivo* organ cells after contacting with the said modulating molecular entities of the MARCH-I, MHC II and/or CD86 amount.

The present invention provides several advantages and solves several relevant technical problems: not only preserves the organ useful life as also starts a process in the organ inhibiting the acute rejection; lengths the useful life *in vivo* of the organ; has local immunomodulation effects in the recipient; generates indirectly a impact on the quality of life of the transplant recipient when requiring less immunosuppressive medications; induces the regulatory T cells activity (which are recognized as immunosuppressive), lowers the MHC molecules expression in the graft, lowers the alloreactive expression in the lymph nodes of the recipient, lengths the graft survival in the recipient.

These and other aspects of the invention are immediately contemplated by the people skilled in the art and by the companies interested on the segment, and will be described in enough details for its reproduction in the following description.

### Brief Description of the Drawings

Figure 1 shows an embodiment of the invention in which the graft treatment with a dose of Hsp70 lengths the cutaneous allograft acceptance in a murine model.
Figure 2A shows the donor cells screening in the recipient DLN 24h or 96h after the transplantation, by the expression of I-A^{b}.
Figure 2B shows the donor cells screening in the recipient DLN 24h or 96h after the transplantation, by the expression of GFP.
Figure 2C shows representative dot plots and percentages of the CD11 c+ cells from donors screened in the recipient DLN by the expression of I-A^{b}.
Figure 2D shows representative dot plots and percentages of the CD11 c+ cells from donors screened in the recipient DLN by the expression of GFP.
Figure 2E shows the expression of CCR7 in the cells I-A^{b}+ screened in the transplanted animals DLN.
Figure 2F shows the expression of CCR7 in the GFP+ population screened in the transplanted animals DLN.
Figure 3 shows the evaluation results of one embodiment of the invention, in which the Hsp70 inhibits the MHC II expression in different DCs.
Figure 4 shows the evaluation results of one embodiment of the invention, in which the Hsp70 partially inhibits the MHC II expression in active BMDCs.
Figure 5 shows the evaluation results of one embodiment of the invention, in which the Hsp70 inhibits the CD86 expression in active BMDCs.
Figure 6 shows the evaluation of one embodiment of the invention, in which the treatment with Hsp70 inhibits the MHC II expression of the donors DCs, and not of the recipients.
Figure 7 shows that the mouse DLN that received grafts treated with Hsp70 showed a reduced proliferation of CD4+ and CD8+ T cells.
Figure 8 shows that the recipients' treatment with BMDCs treated with Hsp70 may length the cutaneous allograft acceptance.
Figure 9 shows the DCs subtypes analysis that were migrating from the graft to the recipient DLN after the graft treatment with Hsp70.
Figure 10 shows the experimental results approving the cells phenotype migrating from the graft to the recipient DLN: CD11 c+ Langerin+ CD11 b-CD103+.
Figure 11 shows that the production of Hsp-induced IL-10 leads to the MARCH-I expression increase and this is fundamental for reducing the MHC II expression.
Figure 12 shows that TLR2 is necessary for producing IL-10, for reducing the MHC II expression and for inhibiting the acute rejection, all induced by Hsp70.
Figure 13 shows the experimental results that ERK and STAT3 are necessary for reducing the Hsp70-induced MHC II expression.
Figure 14 shows that ERK and STAT3 are necessary for reducing the Hsp70-induced MHC II expression.
Figure 15 shows a schematic representation of the signaling route model of the IL-10 production via TLR2-ERK-STAT3.
Figure 16 shows the comparative results among the Hsp70 and the synthesized peptides and the ones tested.
Figure 17 shows that the graft treatment with fragment (peptide) of Hsp70 lengths the cutaneous allograft acceptance in murine model.
Figure 18 shows that the graft treatment with a dose de Hsp70 lengths the cutaneous allograft acceptance in a murine model.

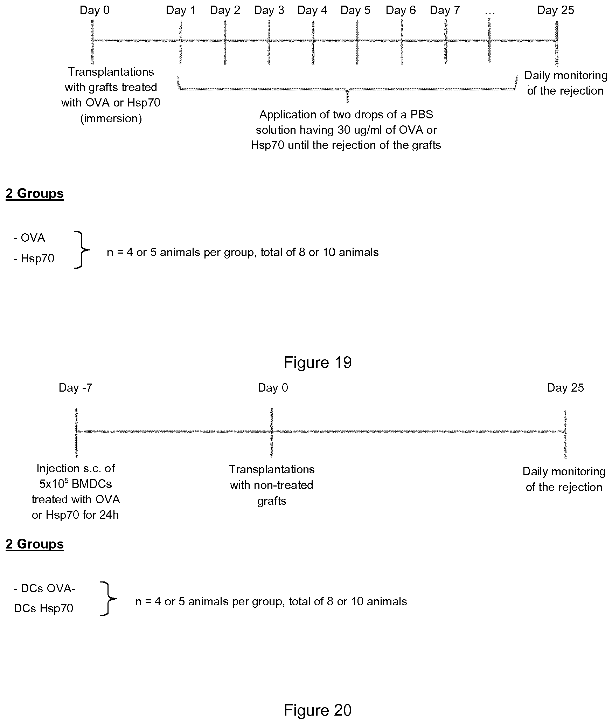

Figure 19 shows the topic treatment until the graft is rejected.
Figure 20 shows the treatment with BMDCs 7 days before the transplantation.

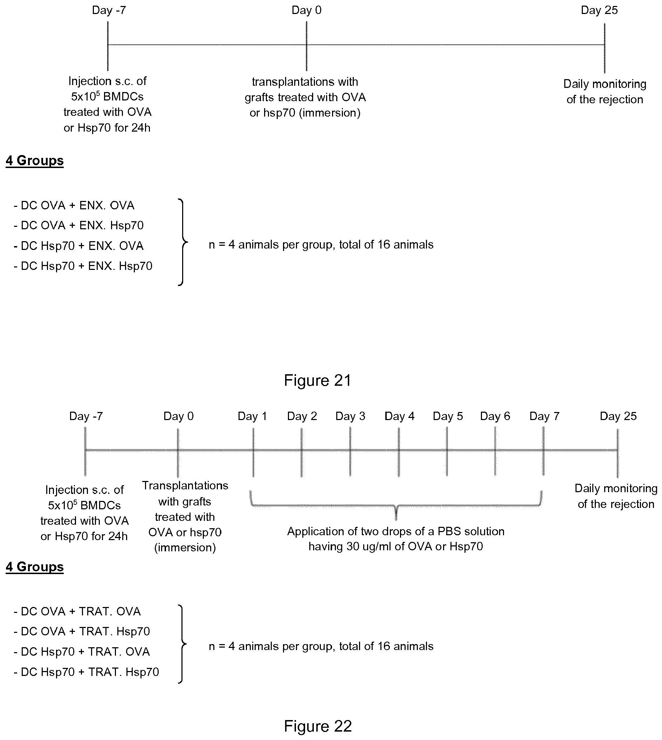

Figure 21 shows the treatment with BMDCs 7 days before the transplantation + immersion of the graft.
Figure 22 shows the treatment with BMDCs 7 days before the transplantation + immersion of the graft + topic application for 7 days.
Figure 23 shows the treatment with BMDCs 7 days before the transplantation + immersion of the graft + topic application until the graft is rejected.
Figure 24 shows the dendritic cells treatment results with Hsp70 in the MARCH-I induction, through analysis of the levels of MARCH-I mRNA through PCR in real time, regarding other immunosuppressant. The β-actin was used as a setter.
Figure 25 shows the LDH amount data in the supernatant (25 A) the amount of released LDH regarding the total LDH present in the cell (25 B), in VERO cells grown for 30h on ice, contacting: 1) the EuroCollins solution; 2) the EuroCollins solution + 30 μg/ml of *M. tuberculosis* Hsp70; 3) the EuroCollins solution + 30 μg/ml of OVA; and 4) PBS 1x (negative control).
Figure 26 shows images of cells confocal microscopy of the renal tissue cell line VERO (ATCC - CCL-81), marked with the kit Live/Dead. Living cells are shown in green and dead cells are shown in red. The cells treated with PBS died much more than when compared with other treatments, after 30h of static storage on ice. There was no difference of cell death occurrence, when compared to EuroCollins and EuroCollins+Hsp70 groups.

### Detailed Description of the Invention

The main function of the organs preservation solutions is protect the organ from cell damage and cell death caused by the long static storage on ice, which may achieve 30h in Brazil. However, the organs preservation solutions known up to now do not solve satisfactorily the technical problems related to the viability maintenance difficulty of transplantation organs and the difficulty of limit its acute rejection after the transplantation. The present invention solves these problems from the MARCH-I, MHC II and/or CD86 modulation in the organ cells already removed from the donor, i.e., *ex-vivo,* instead of modulating the organ recipient person cells, as have been considered the conventional approaches. Besides solving technical problems related to the effective success of the transplantation, once the invention provides rejection reduction, the invention also provides more alternatives to the doctor and to the patient receiving the organ, once it is the organ to be transplanted that is subject to the treatment invention, not the patient receiving it. This approach has substantial benefits and was not available up to now.

For better comprehension of the invention, some definitions will be provided in the following.

### Organs conventional preservation solutions

In the context of the present patent application, the term must be understood as any solution preserving any organ and/or part of the tissues. Also in the patent application context, the term may also be understood as any solution preserving any organ and/or part of animals tissues to be transplanted, including the transplantations either allogeneic as the self-transplantations. The animals comprise mammals, including human beings. Examples of organs preserving conventional solutions are: solution of Winscosin (Viaspan^{®}); Euro-Collins (RENOGRAF^{®}); Histidine-Tryptophan-Ketoglutarate (HTK) solution - Custodiol^{®}; Celsior^{®} Cold Storage Solution, CoStorSol^{®}, MaPerSol^{®}, among others. The description made above of some of them must not be understood as limiting the term to such examples.

### Organs

In the context of the present patent application, the term must be understood as any organ of an animal including mammals and also the human beings. The term "organ or "organs" comprises: skin, kidney, liver, heart, lungs, cells used for bone marrow transplantation, spleen, bones and cartilages, eyes or eyes parts (for example, corneal), among other organs.

### Application of an efficient dose

In the context of the present patent application, the term must be understood as the application of an efficient dose to achieve the biological effect of MARCH-I, MHC II and/or CD86 modulation, not matter if the desired biological effect is achieved only partially (for example, an effect of 10%, 20%, 30%, 50%, 60%, 70%, 80% to 100%). In particular, it is understood that the term refers to the efficient dose application of one or more organ preservation solutions in combination with any molecular entity modulating MARCH-I, MHC II and/or CD86, as for example, the Hsp, or fragments thereof, as mentioned bellow.

### Hsp

In the context of the present patent application, the term must be understood as any protein that is induced by heat or stress, being known also as *heat shock proteins.* The Hsp comprise the proteins Hsp70, Hsp60, Hsp40, Hsp90, Hsp110 and combinations thereof.

### Hsp fragments

In the context of the present patent application, the term must be understood as any peptide sequence (or peptide) derived from a protein Hsp, as Hsp70 (DnaK), heat shock protein achieved from the DnaK sequence of *Mycobacterium tuberculosis.*

### Functional equivalent variations

In the context of the present patent application, the term must be understood as any peptide present in the complete sequence of amino acids of a Hsp, regardless the amino acids chain size, and having the same biological function and/or the same biological effect of MARCH-I, MHC II and/or CD86 modulation. It includes the proteins Hsp70, Hsp60, Hsp40, Hsp90, Hsp110 and also the Hsp70 fragments with peptide sequences represented by SEQ ID No:01, SEQ ID No:02, SEQ ID No:03, SEQ ID No:04, SEQ ID No:05, SEQ ID No:06 or SEQ ID No:07, or also, the complete Hsp70 of *Mycobacterium.*

The invention composition provides the physical environment for achieving the technical effects of the invention. The presence of a MARCH-I, MHC II and/or CD86 modulator in organs preservation compositions provides an alternative approach for preserving the organ and, at the same time, reduce the organ rejection after transplantation. In one embodiment, the invention composition comprises the Hsp70 as MHC II and CD86 modulator in the donor's cells. In addition, this embodiment of the invention composition modulates MARCH-I in the graft DCs, providing the rejection response reduction. It is pointed out that other molecules that are routinely used in the clinic for inhibiting the rejection, as Cyclosporine A and Rapamycin, do not provide the modulation effects of MARCH-I, MHC II and CD86; for the better knowledge of the inventor, however, the invention composition was the first preservation composition of *ex-situ* organs providing such effects in the donor organ.

### Example 1. Composition for ex-vivo preservation and immunomodulation of organ cells to be transplanted. Use of a substance modulating MARCH- I, MHC II and/or CD86: Hsp70 in preservation solution

In this embodiment of the invention, the substance modulating MARCH-I, MHC II and/or CD86 is the *Mycobacterium tuberculosis* Hsp70 (sequence number NC_000962.2 of the GeneBank). This protein was achieved from the coding gene subcloning in the plasmid pET23a, introduced into the strain BL21 of *Escherichia coli.* After bacterial cultivation and production of the protein, this was purified according to Mehlert & Young, using an ATP-agarose (Sigma) column to purify the protein. For determining the protein concentration the flurimetric test Quant-iT™ Protein Assay Kit (Invitrogen) was used and the samples were read in the Qubit® fluorometer (Invitrogen). For removing the lipopolysaccharide (LPS), a method using Triton X-114 (Sigma) described pm Aida and Pabst was used. The contaminant Triton was removed through protein incubation with Biobeads (Bio-Rad) at 4°C stirring for 8h. To check if some contaminant LPS remained in the sample, a bioassay was performed. Mice BALB/c were anaesthetized, intraperitoneally, with 100µL of a solution volume in PBS with 34% of Ketamine and 10% of Xylazine. Later, were injected i.v. with 100 µL of PBS, PBS with 30 µg of LPS (Sigma) or PBS with 30 µg of TBHsp70. After six hours, the animals experienced euthanasia in the CO₂ model, model CO2G, Vol: 30L³, and had the spleen removed. A treatment with D collagenase was done and, subsequently, the cell suspension achieved was analyzed for CD86 expression through flow cytometry, as described on Khoruts and collaborators.

For inhibiting the acute rejection, the graft was soaked into a solution of 500 µL of sterile PBS 1x having 30 µg of TBHsp70, i.e., a solution of 60 µg/mL or 0.06 g/L.

Among the organ preservation solutions used in combination with the Hsp in the present embodiment of the invention composition include:
- Solution of Wisconsin - Viaspan® - manufactured by Organ Recovery Systems;
- Histidine-Tryptophan-Ketoglutarate (HTK) solution - Custodiol® HTK - manufactured by Essential Pharmaceuticals, LLC;
- Celsior® Cold Storage Solution - manufactured by Genzyme;
   and/or
- Solution Euro-Collins - RENOGRAF® - manufactured by Claris Lifesciences Ltd.

### Brief abstract of example 1

In this embodiment, results are shown that present the expression of molecules reduction of the MHC II is caused by the Hsp70, being this the mechanism that inhibits the acute rejection to allografts. Such phenomenon depends on the expression of TLR2 and IL-10 in the graft cells and results in a lower proliferation of CD4+ and CD8+ T cells in the draining lymph node (DLN) of the recipient.

The graft treatment with Hsp70 reduces the MHC II expression exclusively in the dendritic cells (DCs) CD103+, which are able to migrate efficiently to the recipient DLN. Remarkably, the Hsp70 induced an increase in the production of anti-inflammatory cytokine IL-10 by the DCs. This production was mediated by the molecules ERK and STAT3 activation, after engaging the recipient TLR2. In addition, the MHC II expression reduction induced by the Hsp70 coincides with an increase of the MARCH-I ubiquitin molecule expression and both phenomena depended on the molecules TLR2, ERK, STAT3 and IL-10. However, the results not only clarify the mechanism for immunosuppressant effects of the Hsp70, when the said Hsp70 is used in a way to treat grafts and not the grafts recipient people, as also support and enable the process for screening the new molecular entities useful for preparing the composition for *ex-vivo* preservation and/or immunomodulation of organs for transplantation.

### Methodology used in the example 1 experiments

### Used animals

In the embodiment of example 1, the following animals were used: female mice (*Mus musculus*) BALB/c and C57BI/6; knockout animals C57BI/6 for TLR2 (*TLR2*^{*-*/*-*}); wild animals 129Sv/Ev (WT) and knockouts for IL-10 (*il10*^{*-*/*-*}); and C57BL/6-GFP animals. All animals used in the experiments ranged between 6 and 10 weeks old and were kept in individual cages (Techniplast). Autoclaved water and feed were provided freely. All shaving was also autoclaved. The cases maintenance (individual cages) for water change, feed and cleaning were performed twice a week. Animals were maintained in environment with controlled temperature of 22-23°C and light-dark cycles of 12 hours. The keepers always used surgical and masks glove when handling animals. The project was approved by the Ethics Committee for Animals Use of the Pontifical Catholic University of Rio Grande do Sul under registration CEUA 08/00048.

### Protein purification and Lipopolysaccharide (LPS) extraction

For producing the recombinant Hsp70, DnaK gene of *M. tuberculosis* was inserted into a vector pET-23a(+). The plasmid was assembled by the company *Genscript-* USA, the protein was produced in the strain BL21 of *Escherichia coli* and purified according to MEHLERT & YOUNG (1989) teachings.

For removing LPS, a compound was used (1,1,3,3-Tetramethylbutyl)phenol-polyethylene glycol (product Triton X-114, manufacturer Sigma-Aldrich), as described on AIDA & PABST (1990). The contaminant (1,1,3,3-Tetramethylbutyl)phenol-polyethylene glycol was removed though protein incubation with the adsorbent Biobeads (Bio-Rad) at 4°C stirring for 12h. For determining the protein concentration the fluviometric test Quant-iT™ Protein Assay Kit (Invitrogen) was used and the samples were read in the Qubit^{®} fluorometer (Invitrogen).

### Murine model of cutaneous allograft

A method for treating grafts, including allografts, was developed by the inventors of the present invention. Thus, the method treats the grafts (that may be full organs) instead of recipient that will receive the graft, using as tool a classic murine model of skin transplantation and well established. Such method, to the light of the inventors, may be considered as new and inventive. The said model consists of a complete incompatibility of MHC molecules and was described by BILLINGHAM & MEDAWAR (1951). In the said model, the donor mouse C57BI/6 (B6), who expresses the MHC III-A^{b} molecule, is sacrificed and a piece of 1 cm² is removed from the animal tail skin and immersed into a phosphate buffered solution (PBS) having 60 µg/mL of Hsp70 or egg albumin (OVA), for 60 minutes, on ice.

BALB/c recipients, which express the MHC II I-A^{d} molecule, are anaesthetized and the dorsal stem hairs are removed. In the waxed area, 1 cm² of the skin was removed in each recipient mouse and the donor tail skin was sutured in the exposed area. The animals are maintained in individual cages and daily monitored. The graft rejection is confirmed by observing the cyanosis, erythema, erosion and cutaneous graft loss.

### Dendritic cells culture and isolation

Dendritic cells (DCs) were different from the bone marrow of C57BI/6 WT or TLR2 KO mice in the presence of GM-CSF and IL-4 (both achieved from BD Biosciences), as described by INABA and collaborators (2002). The cells were cultivated in 24-well plate in serum free mean, the AIM-V® (Gibco). In the sixth culture day, the non-adherent cells (DCs) were separated from adherent cells. Then, the BMDCs were incubated with 60 μg/ml of Hsp70, 60 μg/ml of OVA, 10 μg/ml of Peptide-glycan - PGN (Sigma-Aldrich) or Hsp70 + 20 ng IL-10 recombinant murine (R&D System) for 24h and, subsequently, analyzed by flow cytometry or had the RNA total extracted. The supernatants were collected and used for analyzing cytokine.

CD11 c+ cells were purified from lymph nodes that drain mice skin antigens C57BI/6 WT or TLR2 KO, and 129Sv/Ev WT or IL-10 KO. The animals were sacrificed and the axial, brachial and inguinal lymph nodes were removed. These organs were macerated in a nylon screen with a mean that has D Collagenase (Roche). The cell suspension achieved was marked with the magnetic beads conjugated with an anti-CD11c antibody (N418) (Miltenyi Biotech) for 15 min. After being washed, the CD11 c+ cells were purified by positive selecting using the MACS MS (Miltenyi Biotech) separation columns, according to the manufacturer instructions. The cells purity purified was controlled by flow cytometry, indicating a purity of 90% CD11 c+ cells in the positive fraction. Therefore, the cells were grown in 96-well plates in serum free mean AIM-V® (Gibco).

DCs were incubated with 30 µg/mL of Hsp70 or OVA for 24 h and after analyzed by flow cytometry or had the RNA totally removed. The supernatants were collected and used for analyzing cytokine. The cells were further treated with 20 ng/ml of Rapamycin (Cell Signaling), 1 μg/ml of Cyclosporine A (Roche) or 20 ng/ml of IL-10 recombinant murine (R&D System) for 24h and the total RNA was removed.

### Evaluation of donors' migratory cells and recipients responses

Cells of the donor mice (I-A^{b}+ or GFP+) were detected in the axillary lymph nodes of the recipients, 24h or 96h after the transplantation by flow cytometry. The transplanted mice were sacrificed, the axillary lymph nodes were removed and macerated in a nylon screen with a mean that had D Collagenase (Roche^{®}). We achieved a cell suspension that had the cells in contact with Tripan blue and analyzed by flow cytometry.

### Intracellular marking

In the sixth culture day, dendritic cells derived from bone marrow (BMDCs) of WT or TLR2 KO mice were stimulated with 30 μg/ml of Hsp70 for 0, 15, 30 or 45 minutes. As positive control, the cells were stimulated with 50 µM of phorbol myristate acetate (PMA) for 30 minutes. After the stimuli in the periods mentioned, the cells were fixed with *Cytofix Buffer* (BD Biosciences) for 10 minutes at 37°C and permeated with *Phosflow Perm Buffer III* (BD Biosciences) for 30 minutes on ice. After such procedures, the cells were marked for p-ERK1/2 and p-STAT3. In addition, BMDCs WT were treated with 30 µM of the selective inhibitor of ERK, PD98059 (Cayman Chemical), for 40 minutes or 50 µM of the inhibitor via JAK2/STAT3, AG490 (Sigma-Aldrich), for 60 minutes or were maintained without treatment, previously to the treatment with the Hsp70.

### Flow cytometry

For analyzing the migration and activation of the DCs subtypes, the cells were marked for I-A^{b} (AF6-120.1), CD11c (HL3), CD11 b (M1/70), CD45R/B220 (RA3-6B2), CD103 (M290), CCR7 (CD197 - 4B12), CD80 (16-10A1), CD86 (GL1) antibodies achieved from BD Biosciences; and Langerin (CD207 - eBioRMUL.2) from eBioscience.

For analyzing the recipients T cells, the cells were marked for CD4 (GK1.5), CD8a (53-6.7), Foxp3 (MF23), Ki67 (B56) achieved from BD Biosciences. The specific transcription factor marking of Tregs Foxp3 was performed using the kit *Mouse Foxp3 Buffer Set* (BD Biosciences), according to the manufacturer instructions. The levels of phosphorylated ERK and STAT3 were determined using antibodies for p-ERK1/2 and p-STAT3 (BD Biosciences) in BMDCs previously fixed and permeablized. The cells were subsequently analyzed in the flow cytometer FACSCanto II (BD Biosciences) with the BD FACSDiva (BD Biosciences) program. The data achieved were analyzed with the Flowjo program (version 7.6.5, Tree Star).

### IL-10 Cytokine measure

The DCs cultures supernatants treated with Hsp70 or OVA for 24h were analyzed regarding the presence of IL-10 with the aid of *CBA Mouse Inflammation* kit (BD Biosciences), according to the manufacturer instructions. The samples were subsequently analyzed in the flow cytometer FACSCanto II (BD Biosciences) with the BD FACSDiva program (BD Biosciences). The data achieved were analyzed with FCAP Array program (version 3.0, BD Biosciences) and expressed in pg/ml.

### Real time PCR

The total RNA was isolated from the DCs treated using the kit RNAeasy (Qiagen), according to the manufacturer instructions. The total purified RNA concentration was determined with the kit Qubit® RNA Assay Kit (Invitrogen), read in the Qubit® Fluorometer (Invitrogen). 50 ng of the RNA had reverse transcriptase with 100U of Sensiscript (Qiagen). DNA concentrations were determined with the kit Qubit® dsDNA HS Assay (Invitrogen), read in the Qubit® Fluorometer (Invitrogen). In a final volume of 10 µL, 8 ng of cDNA were applied using some assays Taqman® Gene Expression (Applied Biosystems): IL10 (Mm00439614_m1), March1 (Mm00613524_m1) and β-actin (4352933E), according to the manufacturer recommendations.

The quantitative real time PCR was performed in the instrument StepOne™ Real-Time PCR System (Applied Biosystems). The relative levels of mRNA were calculated according to the comparative method of Ct, described by SCHMITTGEN & LIVAK (2008). The β-actin constituent gene was used as setter. Non-treated DCs served as reference for OVA or Hsp70-treated DCs.

### Epitopes mapping of T cells

The epitopes mapping of T cells was performed for proving the peptides binding and *in vivo* T cells stimulation. For this purpose, it was used a protocol described in the MacLeod and collaborators (2008) paper. Briefly described, a solution that had 100 µg of TBHsp70 + 7 µg of LPS (served as adjuvant) was injected *i.p* in BALB/c mice. After nine days, the animals experienced euthanasia, spleens were removed and treated with Collagenase D. Splenocytes were plated in a concentration of 10⁶ cells/mL. Cells were stimulated with DMSO (negative control, since it was used for diluting peptides), 60 μg/ml of Hsp70 or 5 μg/ml of each one of the selected peptides, separately for 24h. After this period, the cells were marked for CD11 c-PECy7, CD4-PE-Cy5.5, CD25-PE, Foxp3-APC, B220-APC, Gr1-PE, B220-PerCP and the supernatant analyzed regarding the presence of IL-10, kit CBA Mouse Inflammation (BD Biosciences), according to the manufacturer recommendations. All samples were read in the flow cytometer FACSCanto II (BD Biosciences).

### Statistical analysis

The statistical analysis were performed with the aid of Prism software (version 5.00, Graphpad Software Inc., San Diego). Kolmogorov-Smirnov tests were applied for determining data distribution. In case of distributions that were considered normal, the parameters comparisons between different experimental groups were performed by t test of Student or variance analysis of one way (*one-way* ANOVA) followed by the post-hoc Bonferroni test. In case of abnormal distribution, Kruskall-Wallis and Mann-Whitney tests were used, with Dunn pos-test to discriminate the differences. For graft survival analysis the Kaplan-Meier method was used. The results were expressed on average (normal distribution) or median (abnormal distribution) and average standard deviation and P values lower than 0.05 indicated significant differences.

### Results A single graft treatment with Hsp70 leads to an elongated survival of the graft

For investigating the Hsp70 protector role in the grafts treatment with the protein Hsp70, a new and inventive method was developed for graft treatment, instead of the recipient, using as tool a classic and well established skin transplantation murine model. This model consists of a complete incompatibility of MHC molecules and was described by BILLINGHAM & MEDAWAR (1951). In this model, the donor mouse B6, which expresses the MHC II I-A^{b} molecule, is sacrificed. Then, a piece of 1 cm² was removed from the animal tail skin and immersed into a PBS solution having 60 µg/mL of Hsp70 or OVA, for 60 minutes, on ice. After this period, the grafts were sutured in the BALB/c recipients' back, who express the MHC II I-A^{d} molecule, and the rejection clinical progression is daily monitored.

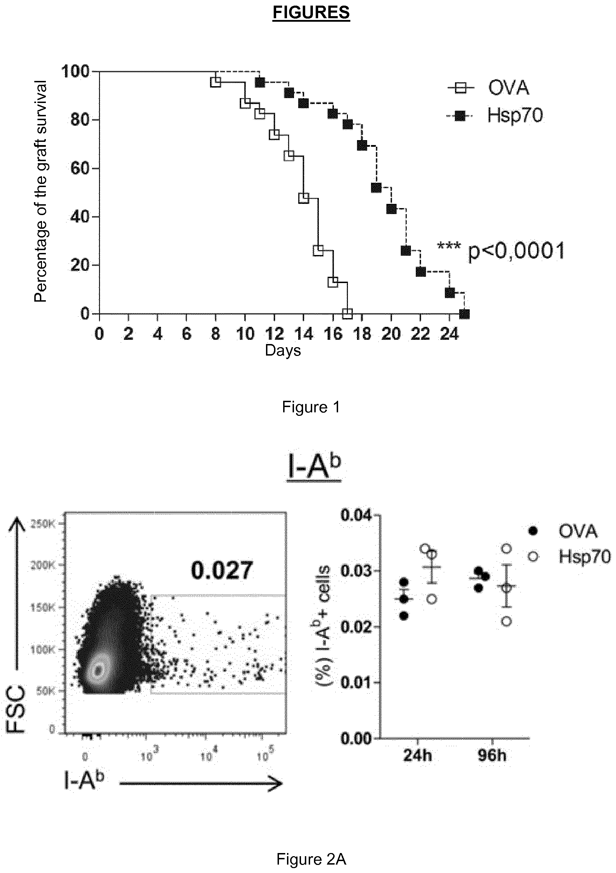

It may be noted in figure 1 that the animals receiving grafts treated with Hsp70 showed a greater survival when compared with the animals that received grafts treated with OVA. Cutaneous grafts of wild animals B6 (I-Ab) (WT) were immersed into a solution having 60 μg/ml of Hsp70 or OVA for 60 minutes at 4°C. After that, the grafts were sutured in the BALB/c (I-Ad) animals' back. The animals were daily monitored. ***, p<0.0001. n= 22-24 mice per group. The results achieved putting the seven independent experiments together.

It may be noted that the survivals of each mouse that received cutaneous grafts treated with OVA or Hsp70. A significant difference was achieved (p<0.0001) when survivals medians of both groups are compared (Table 1).

**Table 1: Effect of the graft immersion-treatment with a dose of Hsp70 or OVA in cutaneous allograft murine model. The results achieved with the seven independent experiments combination.**

| **Treatment** | **Survival (days)** | **Survival average** | **Significance^{a}** |
|---|---|---|---|
| OVA | 8, 10, 10, 11, 12, 12, 13, 13, 14, 14, 14, 14, 14, 15, 15, 15, 15, 15, 16, 16, 16, 17, 17, 17 | 14,0 | |
| Hsp70 | 13, 14, 16, 17, 18, 18, 19, 19, 19, 19, 20, 20, 21, 21, 21, 21, 22, 22, 24, 24, 25, 25 | 20,0 | *** |

| | | | |
|---|---|---|---|
| ^{a} Compared with OVA *** P<0.0001 | | | |

### Dendritic cells of the allograft treated with Hsp70 migrate efficiently for draining lymph node of the recipient

The acute rejection depends on the graft DCs migration for the recipient DLN. In skin transplantations, the inhibition of this migration reduces the recipients' sensitivity. With that, it was investigated if the Hsp70 was preventing the donor cells migration to the DLN.

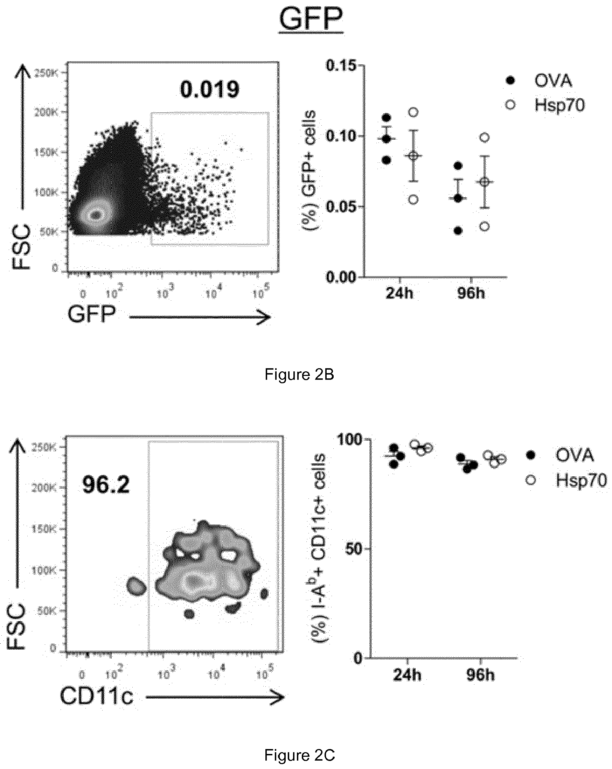

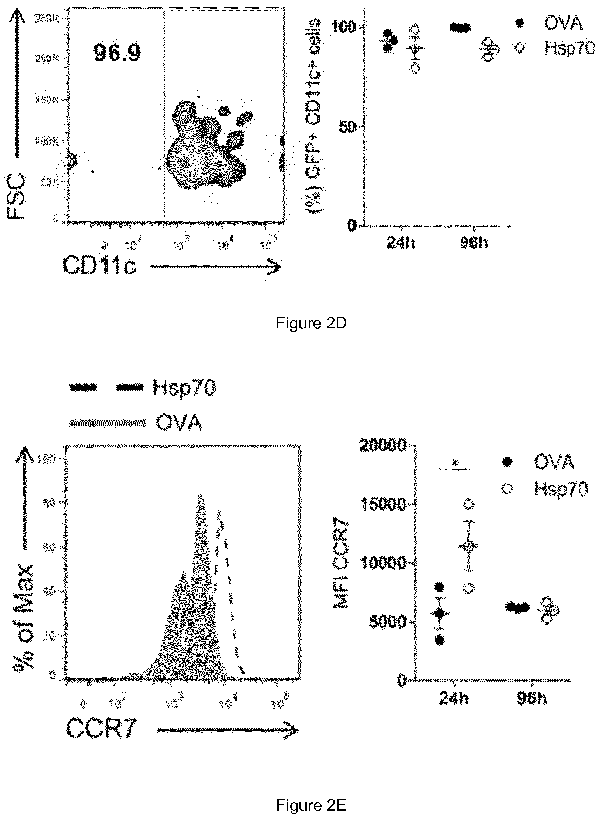

Using the cutaneous allograft model previously described, the graft cells resulted from B6 animals were screened in the BALB/c animals lymph nodes, by two different approaches. Firstly, it was analyzed the presence of cells I-A^{b}+ in the recipient DLN, one and four days after the transplantation. These two moments were chosen since represent both migration moments of the skin cells to the DLN. It was not noted any difference between the mice that received grafts treated with OVA or Hsp70 (Figure 2A). In addition, it was verified that most of migratory cells were DCs (Figure 2C). Then, due to the fact that Hsp70 may modulate the MHC II cells in the DCs, transplantations were repeated using B6 grafts expressing the green protein GFP (*green fluorescent protein)* and the donor cells were screened in the BALB/c recipients. Once again, the donor cells percentage in the recipient DLN did not differ between both treatments (Figure 2B and Figure 2D).

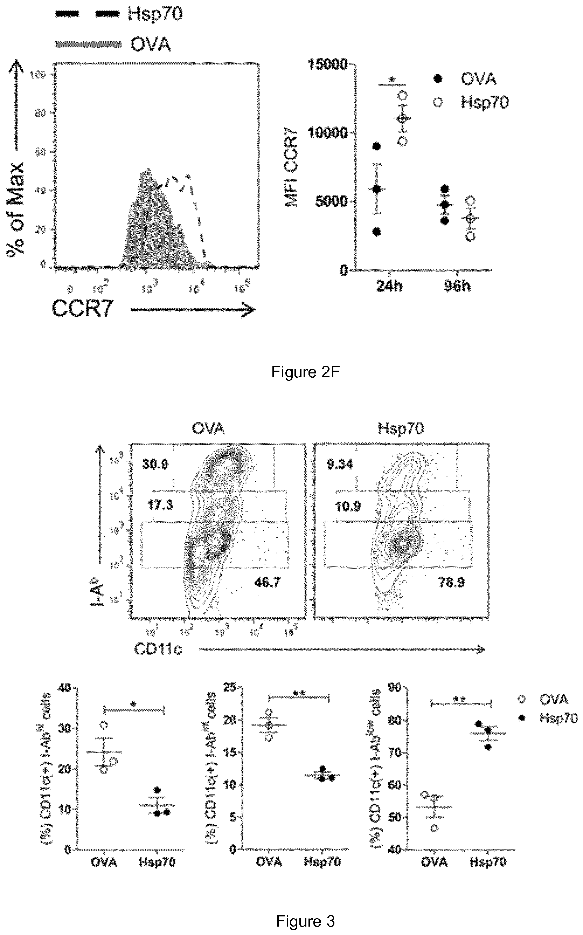

The CCR7 expression is needed for DCs migration to the secondary lymphoid organs. Consequently, the CCR7 expression was evaluated in the donor cells, which were present in the recipient DLN, resulted from grafts treated with Hsp70 or OVA. Surprisingly, after 24 hours from the transplantation, it was noted that the Migratory DCs of grafts treated with Hsp70 expressed upper levels of CCR7 when compared with DCs of the grafts treated with OVA (Figure 2E and 2F).

Four days after the transplantation, the CCR7 levels did not differ between Hsp70 and OVA groups. The data demonstrates that the Hsp70 does not influence the donor DCs migration to the recipient DLN, as showed in figures 2A, 2B, 2C, 2D, 2E and 2F.

In Figures 2A, 2B, 2C, 2D, 2E and 2F, DCs of grafts treated with Hsp70 migrate efficiently to the Recipients DLN. Cutaneous grafts of B6 (I-A^{b}) wild animals (WT) or GFP were immersed into a solution having 60 μg/ml of Hsp70 or OVA for 60 minutes at 4°C. Then, the grafts were sutured in the BALB/c (I-Ad) animals' back. Donor cells were screened in the recipient DLN 24h or 96h after the transplantation, by the expression of (A) I-A^{b} or (B) GFP. (C, D) Representative dot plots and CD11 c+ cells percentages of the donors screened in the recipient DLN. (E) CCR7 expression in the I-A^{b}+ or (F) cells in the GFP+ population screened in the transplanted animals DLN. Graphics expressed on average ± SEM. *, p<0.05. n= 3 mice per group. Representative results of six independent experiments.

### The Hsp70 reduced the MHC II expression and CD86 in activated dendritic cells

The group of inventors found previously that the Hsp70 can delay the *in vitro* differentiation of Mice bone marrow (BMDCs) DCs, which was evidenced by inhibiting the MHC II and the CD86. However, if the Hsp70 could inhibit, the MHC II expression in DCs already differentiated needed to be determined. For answer this question, DCs were isolated from lymph nodes draining antigens resulted from the skin and treated with OVA or Hsp70. The treatment with Hsp70 led to an increase in the number of DCs expressing intermediate or low levels of MHC II when compared with DCs treated with OVA (Figure 3).

DCs isolated from skin draining lymph nodes were treated with OVA or Hsp70 for 24h. After this period, the expression of I-A^{b} in the DCs was analyzed by flow cytometry.

DCs analyzed in Figures 2A, 2B, 2C, 2D, 2E and 2F, instead of different, were found in *steady-state,* not being activated. However, when a transplantation was performed, it is known that there is a local sterile inflammation leading to these cells activation. For determining if the Hsp70 may modulate already activated DCs, the BMDCs were treated with LPS for 24h and after treated with Hsp70 for additional 24h.

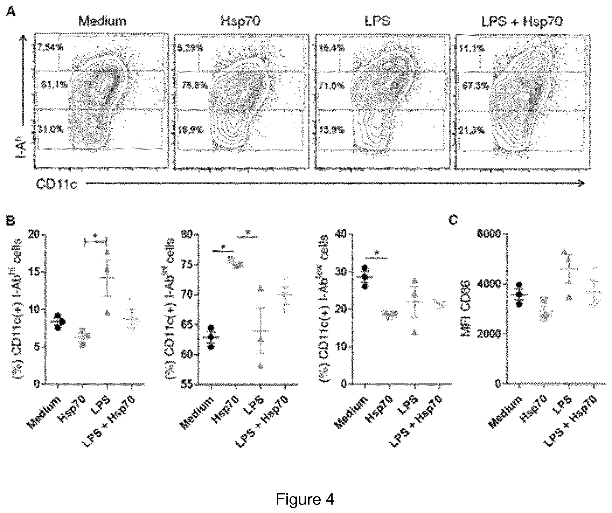

As expected, the group treated with LPS showed a higher percentage of cells expressing high amounts of MHC II (CD11 c+ I-Ab^{hi}) (Figure 4). However, the subsequent treatment with Hsp70 (group LPS+Hsp70) was able to revert partially this progress, being very similar to cells treated only with Hsp70 (Figure 4). DCs were different from the bone marrow of B6 WT mice and in the sixth day of culture were treated or not with LPS for 24h. After this period, these cells were treated or not with Hsp70 for additional 24h. Subsequently, the expression of I-A^{b} was analyzed by flow cytometry. (A) Cells strategy analysis according to the expression of I-A^{b} (*high, intermediate* or *low*)*.* (B) Percentages of CD11 c+ I-Ab^{hi}, CD11c+I-Ab^{int} and CD11 c+ I-Ab^{low} cells. (C) *Mean Fluorescence Intensity -* MFI of I-A^{b} in the CD11 c+ cells. Graphic expressed on average ± SEM. *, p<0.05. Experiments performed in triplicate. Representative of two independent experiments, being the results indicated in Figure 4.

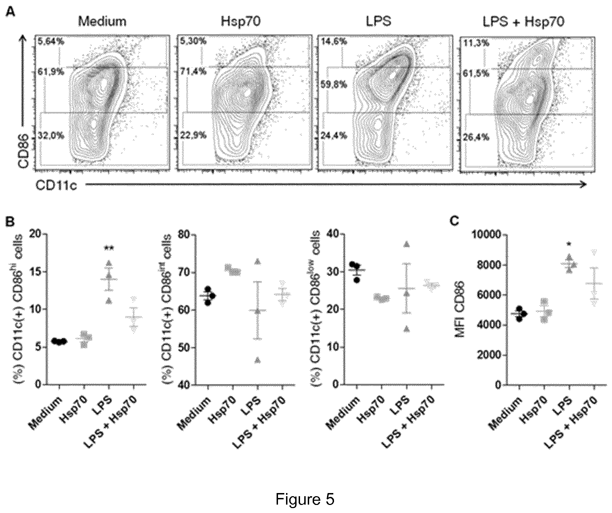

A very similar response could be noted regarding the CD86 expression, in which the treatment with LPS led to a higher percentage of cells expressing high amounts of CD86 (CD11 c+ CD86^{hi}), being the treatment with Hsp70 able to revert partially this expression (Figure 5).

DCs were different from the bone marrow of B6 WT mice and in the sixth day of culture were treated or not with LPS for 24h. After this period, these cells were treated or not with Hsp70 for additional 24h. Subsequently, the CD86 expression was analyzed by flow cytometry. (A) Cells strategy analysis according to CD86 expression (*high, intermediate* or *low.* (B) Percentages of CD11 c+ CD86^{hi}, CD11c+CD86^{int} and CD11c+CD86^{low} cells. (C) *Mean Fluorescence Intensity* (MFI) of CD86 in the CD11 c+ cells. Graphic expressed on average ± SEM. *, p<0.05. Experiments performed in triplicate. Representative of two independent experiments, being the results indicated in Figure 5.

### The treatment with Hsp70 inhibits the direct way, but not the indirect, of the alloantigen recognition by reducing the MHC II expression by donors DCs

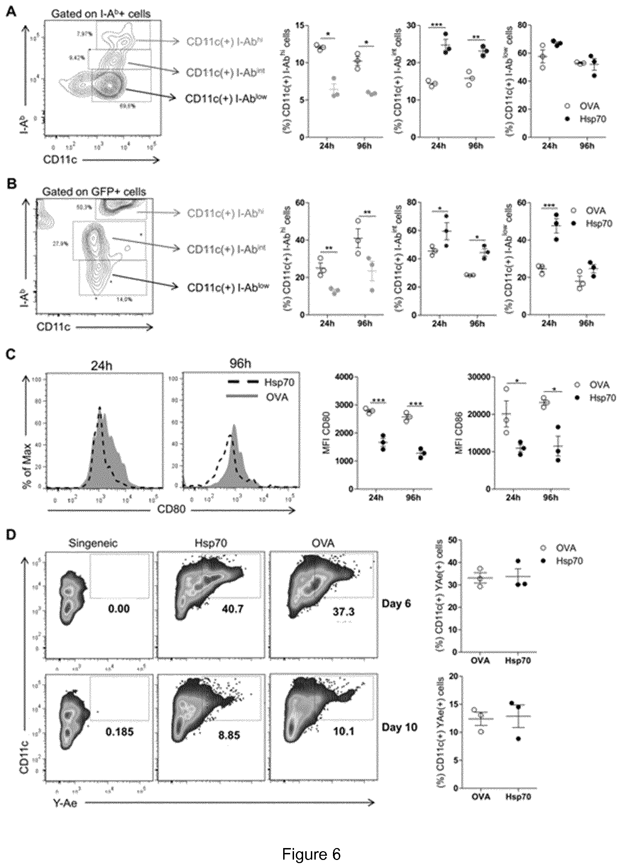

In the direct way of alloantigen recognition, the recipient T cells recognize the donor MHC molecules presenting allopeptides, whereas in the indirect way, the T cells recognize the alloantigens presented in proper MHC molecules. Therefore, the simpler explanation for inhibiting the acute rejection achieved in our system may be because grafts DCs treated with Hsp70 are expressing low levels of MHC II and CD86, inhibiting the direct way of alloantigen recognition. For investigating this issue, levels of I-A^{b} and of co-stimulating molecules in the donors' cells migrating to the recipient DLN were analyzed. In fact, the treatment with Hsp70 led to a MHC II expression reduction in the donors DCs. This was verified with two approaches used previously, through the expression of I-A^{b} (Figure 6, item A) and GFP (Figure 6, item B). We also noted that, 24h or 96h after the transplantations, DCs resulted from grafts treated with Hsp70 showed reduced levels of CD80 and CD86 when compared to DCs of grafts treated with OVA (Figure 6, item C).

For investigating if the Hsp70 may modulate the indirect way of the alloantigen recognition, transplantations were performed with BALB/c animals grafts and the same animals were sutured in B6 recipients. Subsequently, the alloantigens presentation was analyzed by the recipient DCs using the antibody Y-Ae. This antibody recognizes a peptide (Eα₅₂₋₆₈) expressed in BALB/c animals bound in the MHC II I-A^{b} molecule (expressed in the B6 animals). The results showed in Figure 6, item D, disclose that there was no difference in the antigens presentation levels between DCs of the recipients that received grafts treated with Hsp70 or OVA. These findings suggest that the MHC II expression modulation occurs in the donors DCs, and not in the recipients ones. Also, the treatment with Hsp70 inhibits the acute rejection by acting in the direct way of the alloantigen recognition.

In Figure 6: (A) Cells strategy analysis of B6 WT donors, according to the expression of I-A^{b} (*high, intermediate or low)* and percentage of the donors' cells CD11c+I-Ab^{hi}, CD11c+I-Ab^{int} and CD11 c+ I-Ab^{low} in animals that received grafts treated with OVA or Hsp70. (B) Identical to **A,** however donors' cells were screened by the expression of GFP. (C) Representative histogram graph of the CD80 expression in the donors' cells and mean fluorescence intensity (MFI) of CD80 and CD86 in the cells GFP+CD11 c+, 24h or 96h after the transplantation. Graphs expressed on average ± SEM. *, p<0.05; **, p<0.01; ***, p>0.001. n= 3 animals per group. Representative of three independent experiments.

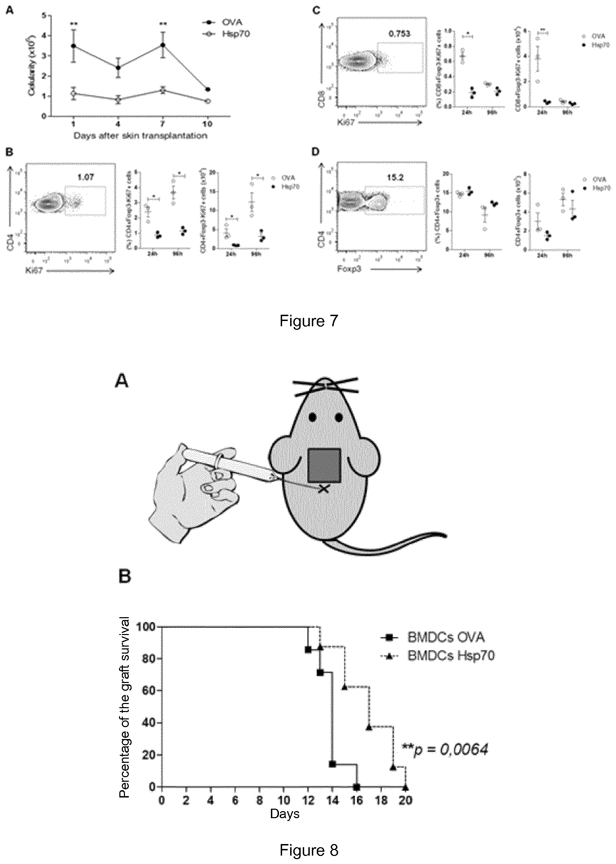

When analyzed the median responses in the Recipients' DLNs, it was noted, for inventors surprise, which animals that received grafts treated with OVA presented organs in a greater size when compared with animals that received grafts treated with Hsp70. For confirming this data, we count the total of cells of the Recipients' DLNs in the days 1, 4, 7 and 10 after the transplantations. In fact, the animals that received grafts treated with Hsp70 had a lower absolute number of cells in all verified days (Figure 7, item A).

The rejection to grafts depends on the CD4+ and CD8+ alloreactive T cells proliferation, in the recipient DLN and probably a lower proliferation of these cells may be led to the lower number of the total of T cells present in the DLNs of animals that received grafts treated with Hsp70. For analyzing this hypothesis, the proliferation of these cells was analyzed by marking with Ki67 in the transplanted animals DLN. Ki67 is a molecule having a role in the cellular cycle and is expressed by any organism cell that is proliferating. It was noted that the graft treatment with Hsp70 leads to a reduction marked in the CD4+ and CD8+ T cells proliferation in the recipient mouse DLN (Figure 7, items B and C). An increase in the number of regulating T cells (Tregs) may reduce the alloreactive T cells proliferation in the recipient DLN, delaying the rejection to grafts. Due to the fact that the inventors' research group has noted previously that the acute rejection inhibition induced by the Hsp70 depends on Tregs, it was investigated if the graft treatment with Hsp70 leads to an increase of the Tregs in the Recipients DLN. Interestingly, it was not detected a significant increase of the Tregs in the recipient DLN after receiving a graft treated with Hsp70 (Figure 7, item D). Our results suggest that the MHC II modulation by the Hsp70 leads to the alloreactive proliferation reduction and not through Tregs. These findings are in accordance with the observation that immature DCs administration in human leads to an inhibition of the CD8+ T cells effector functions.

Figure 7. (A) Count of the DLNs cells, in the days 1,4,7 and 10 after the transplantations, of the recipients that received grafts treated with OVA or Hsp70. (B) Representative dot plot (left panel), percentages (central panel) and absolute number of CD4+Foxp3-Ki67+ T cells (right panel), CD8+Foxp3-Ki67+ T cells (C), or Tregs CD4+Foxp3+ (D). Graphs expressed on average ± SEM. *, p<0.05; **, p<0.01. n= 3 mice per group. Representative results of 3-4 independent experiments.

For confirming that the effect seen by the graft treatment with Hsp70 is due to its action on the donor DCs, grafts cutaneous transplantations resulted from mice B6 and sutured in BALB/c recipients were performed. In this case, grafts were not treated. Instead, B6 mice BMDCs, previously treated with Hsp70 or OVA for 24h, were injected by the subcutaneous way below the sutured graft (Figure 8, item A). The animals were monitored daily for grafts rejection verification. With that, it was attempted to mimic what was happening in the experiments previously showed. As can be noted in Figure 8, item B, and in the Table 3, the animals that received BMDCs from donors treated with Hsp70 presented a higher survival of the graft, at a level similar presented by the animals that received grafts treated with Hsp70 (Figure 1 and Table 2).
Figure 8 shows that the recipients treatment with BMDCs treated with Hsp70 may length the cutaneous allograft acceptance. Cutaneous grafts of B6 wild animals (I-A^{b}) (WT) were transplanted in the BALB/c (I-Ad) animals' back. Grafts did not experience any treatment. Thus, after the grafts are sutured the animals received subcutaneously 10⁵ BMDCs previously treated with 30µg/mL of Hsp70 or OVA for 24h. Animals were monitored daily regarding grafts rejection. n= 7-8 mice per group. Results achieved combining two independent experiments.

**Table 2: Effect of the treatment with 10⁵ BMDCs pre-treated with Hsp70 or OVA in cutaneous allograft murine model. Results achieved combining two independent experiments.**

| **Treatment** | **Survival (days)** | **Survival median** | **Significance^{a}** |
|---|---|---|---|
| BMDCs OVA | 12, 13, 14, 14, 14, 14, 16 | 14,0 | |
| BMDCs Hsp70 | 13, 15, 15, 17, 17, 19, 19, 20 | 17,0 | * |

| | | | |
|---|---|---|---|
| ^{a} Compared with BMDCs OVA ^{*} P<0.0194 | | | |

### DCs CD103+ from donors have the main migratory subtype measuring the tolerance to grafts treated with Hsp70

Three DCs subtypes may be found in the skin: Langerhans Cells (or LCs - Langerin+ CD103- CD11 b+) found in the epidermis; two additional subtypes comprising DCs CD103+ (Langerin+ CD103+ CD11 b-); and "classic" dermal DCs (Langerin- CD103- CD11 b+). These DCs subtypes in the skin have different stimulating abilities, causing the differential polarization of specific T cells for the same antigens. While the LCs may induce Tregs in a homoeostatic state and inflammatory T cells in inflammation conditions, the DCs CD103+ cause the tolerance in the intestine. DCs CD141^{hi} from humans are equivalent to DCs CD103+ from mice. In addition, these cells may inhibit the graft disease against the host in a xenograft model, through IL-10 production.

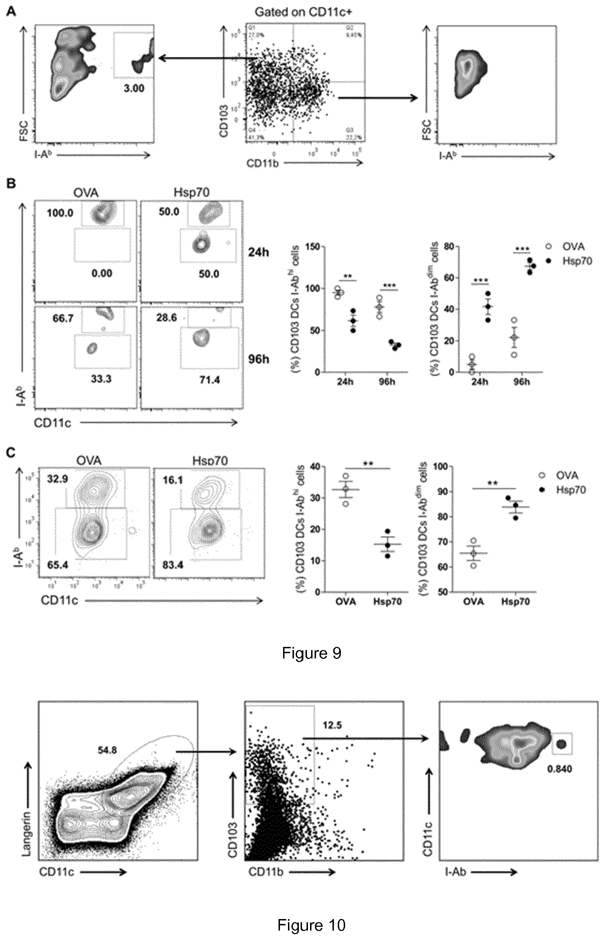

It was investigated which DCs subtype was mediating the tolerance in the system used and, for such effect, the DCs subtypes that were migrating from the graft to the recipient DLN after the graft treatment with Hsp70 were analyzed. It was noted that DCs CD103+ was the only Migratory DCs subtype present in the DLN, either 24h or 96h after the transplantation (Figure 9, item A). In the analyzed periods, the LCs or "classic" dermal DCs migration was not detected. This observation is according to previous studies suggesting that LCs are not necessary for cutaneous grafts rejection with total incapability of MHC; instead, these cells would remain secured in the graft epidermis after the transplantation. In addition, it was noted that there was a significant reduction of MHC II levels in these DCs CD103+ migrating from the graft treated with Hsp70 to the recipient DLN, 24h or 96h after the transplantation (Figure 9, item B). Hsp70 was also able to reduce *in vitro* the MHC II expression in DCs CD103+ isolated from the lymph nodes (Figure 9, item C).

In Figure 9, the DCs CD103+ represent the main cells subtype that arrive in the recipient DLN and may be modulated by the Hsp70. (A) Analysis strategy for DCs subtypes present in the mice recipients DNL. (B) Representative graphs of the DCs CD103+ from donors (analyzed as in A) and percentage of DCs CD103+ expressing high and reduced levels of I-A^{b} in the DLNs of the mice that received grafts treated with OVA or Hsp70. (C) DCs were isolated from LNs draining skin antigens and these cells were treated with 30 μg/ml de Hsp70 or OVA for 24h. After this period, the expression of I-A^{b} was analyzed in the DCs CD103+. Graphs expressed on average ± SEM. **, p<0.01; ***, p>0.001. n= 3 mice per group. Representative results of 3 independent experiments.

It was confirmed in a separated experiment the cells phenotype migrating from the graft to the recipient DLN: CD11 c+ Langerin+ CD11b- CD103+ (Figure 10). In Figure 10, the Migratory DCs from donors are CD11 c+ Langerin+ CD11 b-CD103+. Alternative strategy for DCs CD103+ analysis from the donors present in the BALB/c mice DLN that received grafts from B6 animals WT. These cells were screened with the expression of I-A^{b}.

### The cytokine IL-10 is necessary for reducing the MHC II expression in the DCs and for acute rejection inhibition induced by Hsp70

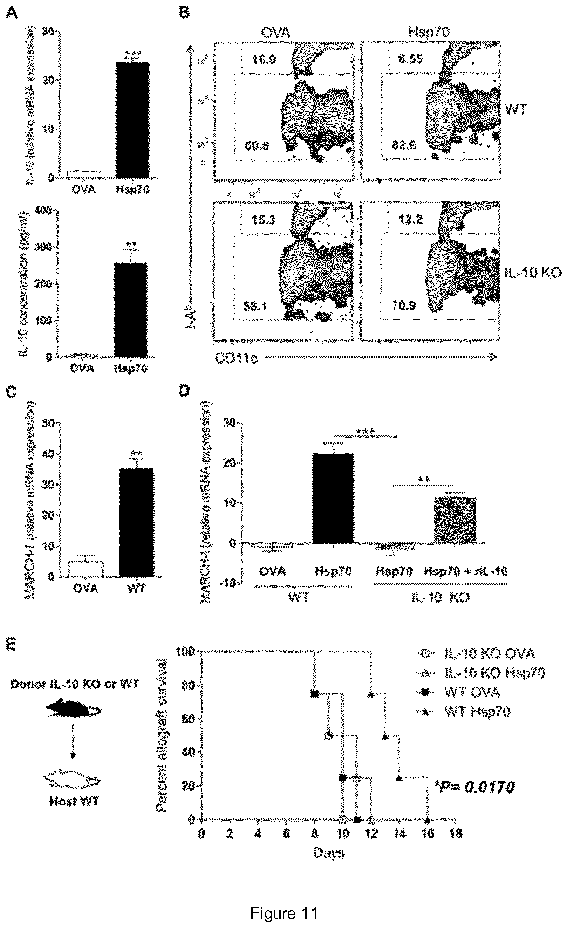

Subsequently, it was analyzed for which mechanisms the Hsp70 would be inducing the MHC II expression reduction in the DCs and subsequently lengthing the graft acceptance. The IL-10 is the main anti-inflammatory cytokine and it is known that it may lead to the MHC II expression reduction. It was demonstrated that Hs70 may induce the IL-10 expression in different DCs from the bone marrow (BMDCs), as well as in arthritis models. Thus, this chaperone exerts its anti-inflammatory effects dependent on the IL-10. It was hypothesized that the IL-10 induction would be the mechanism addressing the MHC II expression reduction in the Stimulating DCs with Hsp70. In fact, the Hsp70 may induce the IL-10 production, either at mRNA level as protein level, in DCs isolated from mice lymph nodes mice (Figure 11, item A).

In addition, the IL-10 expression by these cells was necessary for reducing the Hsp70-induced MHC II expression, once it was not possible to note this phenomenon in knockout animals (KO) DCs for the IL-10 treated with Hsp70 (Figure 11, item B).

Recently, it was identified a regulation mechanism of the MHC II expression in response to the IL-10: the transmembrane protein induction MARCH-I. MARCH-I is a member of the family E3 of ubiquitin ligase and proved to be able of regulating the expression of some molecules as the MHC II, MHC I, CD86, CD4 in the antigen-presenting cells (APCs). When the MARCH-I expression is increased in the APCs, this leads to an ubiquitination and a subsequent degradation of the MHC II and of the CD86. In order to determine if the treatment with Hsp70 may induce the MARCH-I expression in the DCs, DCs were treated isolated from LNs of mice with Hsp70 or OVA and mRNA levels of the MARCH-I were analyzed through a Real-time PCR. It was detected in the DCs an increase of 20 times in the MARCH-I mRNA after the treatment with Hsp70 (Figure 11, item C). For testing if this increase was being triggered by the Hsp70-induced IL-10 production, the DCs isolated from knockout animals for IL-10 were treated with Hsp70 and subsequently the MARCH-I levels of mRNA were analyzed. The DCs IL-10 KO treated with Hsp70 did not present an increase in the MARCH-I levels, when compared with DCs WT (Figure 11, item D). In addition, the addition of recombinant murine IL-10 in the culture reestablished partially the MARCH-I expression by the DCs KO (Figure 11, item D).

However, the production of Hsp70-induced IL-10 leads to the MARCH-I expression increase and this is fundamental for reducing the MHC II expression. An interpretation of these findings was that the IL-10 would be required for mediating the rejection inhibition induced by the Hsp70. For testing this hypothesis, we performed cutaneous transplantations (as described above) using grafts resulted from donors IL-10 KO, treated with Hsp70 or OVA. The absence of IL-10 in the graft cancelled the protection mediated by the Hsp70 (Figure 11, item E).

Figure 11 shows that the Hsp70-induced IL-10 production leads to the MARCH-I expression increase and this is fundamental for reducing the MHC II expression. (A) DCs isolated from LNs draining skin antigens were treated with OVA or Hsp70 for 24h. After that, IL-10 mRNA levels were analyzed by Real-time PCR. The β-actin was used as a setter. The IL-10 concentration was measured in the cultures supernatant by flow cytometry. (B) DCs isolated from LNs draining skin antigens ofWT animals or IL-10 KO were treated with OVA or Hsp70 for 24h and the expression of I-A^{b} was analyzed by flow cytometry. (C) As in A, however the MARCH-I mRNA levels were analyzed by Real-time PCR. (D) DCs isolated from LNs draining skin antigens of WT animals or IL-10 KO were treated with OVA or Hsp70. DCs IL-10 KO were also treated with Recombinant IL-10. All treatments were performed for 24h and MARCH-I mRNA levels were analyzed by Real-time PCR. (E) Cutaneous grafts of WT animals or IL-10 KO were immersed into a solution having 60 μg/ml of Hsp70 or OVA for 60 minutes at 4°C. After this period, grafts were sutured in the BALB/c animals' back and the acceptance was daily monitored. Graphs expressed on average ± SEM. *, p<0.05; **, p<0.01; ***, p>0.001. n= 3-5 mice per group. Representative results of 3 independent experiments.

### The Hsp70 induces the MHC II reduction using the TLR2-ERK-STAT3-IL-10 way

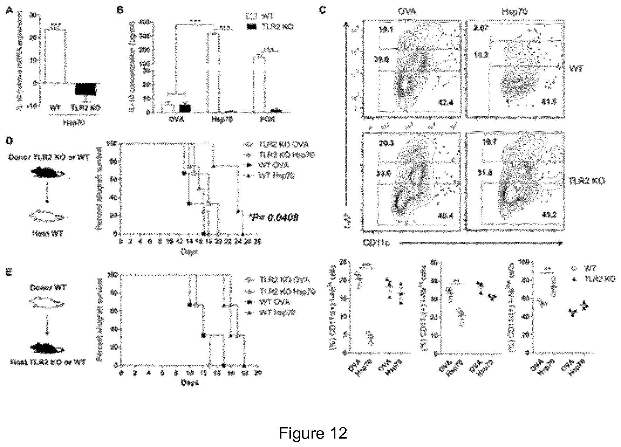

Once determined that the IL-10 is fundamental for protecting effect of the Hsp70 no graft, it was attempted to identify the signaling way that were inducing the IL-10 production in the system. The IL-10 expression regulation was recently associated to the TLR2 membrane recipient, besides inducing the tolerance in diabetes and arthritis. A hypothesis was created that the Hsp70-induced IL-10 expression is mediated by the TLR2. DCs isolated from lymph nodes of WT mice or knockouts for TLR2 (TLR2 KO) are thus treated with Hsp70 or OVA and the IL-10 expression was analyzed, either in mRNA levels as protein. DCs TLR2 KO treated with Hsp70 showed a significant reduction of the mRNA expression (Figure 12, item A) and of the IL-10 protein (Figure 12, item B), when compared with the DCs WT. In addition, the Hsp70-induced MHC II expression inhibition was also dependent on TLR2 in DCs isolated from LNS (Figure 12, item C).

It was also investigated if the TLR2 expression by the donor's cells was necessary for the Hsp70-induced acute rejection inhibition. B6 grafts WT or TLR2 KO were transplanted, immersed into a solution having OVA or Hsp70, in BALB/c recipients. The lengthing of the graft survival in function of the treatment with Hsp70 was dependent on the TLR2 expression in the graft. (Figure 12, item D). To confirm that the Hsp70 was acting in the donor's cells, instead the recipient, cutaneous BALB/c animals grafts WT were transplanted in B6 WT or TLR2 KO recipients. WT grafts treated with Hsp70 showed a very similar survival when transplanted in WT or TLR2 KO recipients (Figure 12, item E), indicating that the Hsp70 is not acting in the recipients' cells modulation.

Figure 12: The TLR2 is necessary for the IL-10 production, MHC II expression reduction and acute rejection inhibition, all induced by Hsp70. (A) DCs isolated from LNs draining skin antigens of WT animals or TLR2 KO were treated with OVA or Hsp70 for 24h and IL-10 mRNA levels were analyzed by Real-time PCR. The β-actin was used as setter. (B) WT or TLR2 KO BMDCs were treated with OVA, Hsp70 or PGN for 24h and the IL-10 concentration was measured by flow cytometry from the supernatant. (C) Representative graphs and percentages of the CD11 c+ I-Ab^{hi}, CD11c+I-Ab^{int} and CD11c+I-Ab^{low} cells of DCs isolated from LNs draining skin antigens of WT animals or TLR2 KO. (D) Cutaneous grafts of WT animals or TLR2 KO were immersed into a solution having 60 μg/ml of OVA or Hsp70 for 60 minutes, on ice. After this period, the treated grafts were sutured in the BALB/c animals' back and the acceptance was daily monitored. (E) As in D, however the donors were BALB/c WT and B6 WT or TLR2 KO recipients. Graphs expressed on average ± SEM. *, p<0.05; **, p<0.01; ***, p>0.001. n= 3-4 mice per group. Representative results of 3 independent experiments.

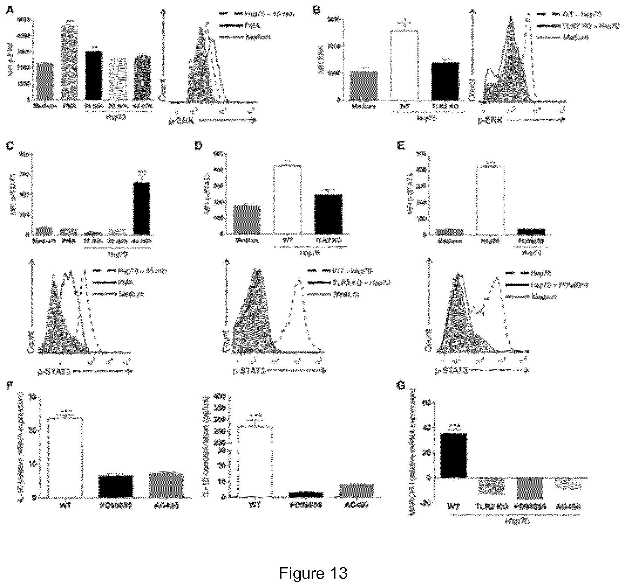

Subsequently, molecular events were characterized that would be subsequent to the TLR2 engagement in the IL-10 production way after the stimuli with Hsp70. The ERK is the main molecule involved in the IL-10 expression by different cells of the immune system. The IL-10 production via ERK after the TLR2 engagement was recently reported. When the BMDCs were treated with Hsp70, it was noted an increase in the phosphor ERK levels (p-ERK) by these cells, after 15 minutes of stimulus (Figure 13, item A). This increase was not noted when the BMDCs treated with Hsp70 were TLR2 KO (Figure 13, item B). Beside the ERK, an important transcription factor that is associated and bound to the IL-10 production and to the tolerance to transplantations is STAT3.

It was previously demonstrated that the Hsp70 may activate the STAT3 in *myeloid-derived suppressor cells -* MDSCs, leading to an immunosuppression. In the studies performed by the inventors' research group, the Hsp70 induced the phosphorylation STAT3 in the BMDCs, 45 minutes after the stimulus (Figure 13, item C). This phosphorylation was dependent on the TLR2 expression in the treated BMDCs (Figure 13, item D).

For verify if the ERK activation was bellow the STAT3 in this way, the ERK expression in the BMDCs was inhibited using the inhibitor PD98059. The ERK inhibition in these cells removed the STAT3 activation against the stimulus with Hsp70 (Figure 13, item E). It was also noted that, either ERK as STAT3, were necessary for Hsp70-induced IL-10 production (Figure 13, item F). In addition, the Hsp70-induced MARCH-I expression increase was dependent on the TLR2-ERK-STAT3 way (Figure 13, item G).

Figure 13: The Hsp70 activates the ERK-STAT3 via TLR2, leading to IL-10 production reduction and the MARCH-I expression increase. In (A): BMDCs were treated with PMA for 30 minutes; Hsp70 for 15, 30 or 45 minutes; or were left without stimulus. The p-ERK expression was measured by flow cytometry. (B) The MFI and representative histogram of the p-ERK expression in the BMDCs of mice WT or TLR2 KO that were treated with Hsp70 for 15 minutes or that were left without stimulus. (C) BMDCs were treated with PMA for 30 minutes; Hsp70 for 15, 30 or 45 minutes; or were left without stimulus. The p-STAT3 expression was measured by flow cytometry. (D) The MFI and representative histogram of the p-STAT3 expression in the BMDCs of mice WT or TLR2 KO that were treated with Hsp70 for 45 minutes or that were left without stimulus and (E) in the BMDCs that had or not the ERK inhibited with the PD98059, previous to the stimulus with Hsp70. (F) BMDCs were treated for 60 minutes with PD98059 (ERK) or AG490 (JAK2/STAT3) inhibitors previous to the stimulus with Hsp70 for 24h. After that, the IL-10 mRNA levels were measured by Real-time PCR and the concentration of this cytokine by flow cytometry, using the kit CBA Mouse Inflammation. (G) BMDCs WT or TLR2 KO were treated for 60 minutes with the PD98059 (ERK) or AG490 (JAK2/STAT3) inhibitors previous to the stimulus with Hsp70 for 24h. After that, the MARCH-I mRNA levels were analyzed by Real-time PCR. The β-actin was used as setter. Graphs expressed on average ± SEM. *, p<0.05; **, p<0.01; ***, p>0.001. n= 3 mice per group. Representative results of 3 independent experiments.

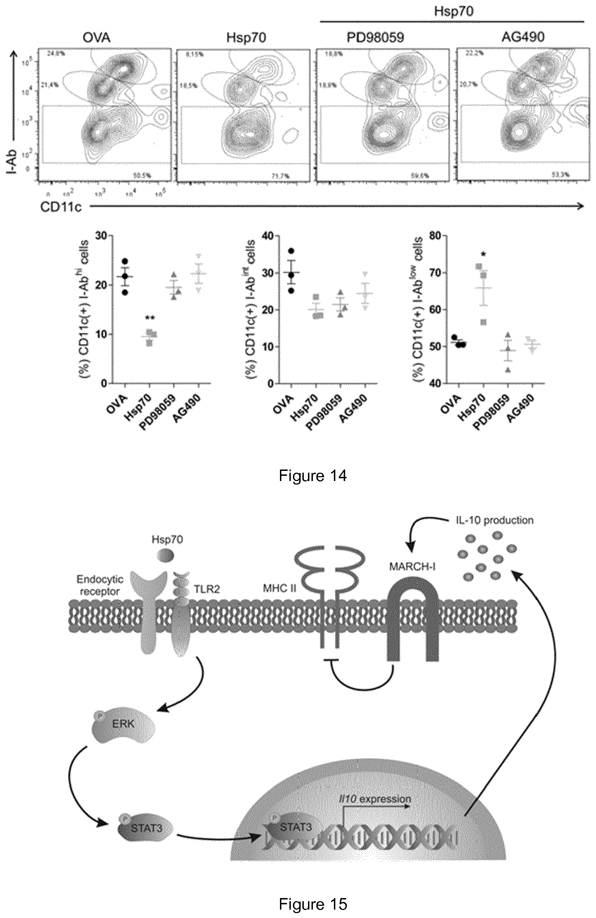

Besides the IL-10 production induction, it was showed that ERK and STAT3 are necessary for reducing the MHC II expression in DCs isolated from Murine LNs and stimulated with Hsp70 (Figure 14). The DCs that had the ERK or the STAT3 inhibited were not able to reduce the MHC II expression after the stimulus with Hsp70 (Figure 14).

Figure 14. ERK and STAT3 are necessary for reducing the Hsp70-induced MHC II expression. DCs isolated from LNs draining skin antigens were treated with the ERK (PD98059) inhibitor or JAK2/STAT3 (AG490) for 60 minutes and then were stimulated with Hsp70 for 24h. After this period, these cells were analyzed for the expression of I-A^{b} by flow cytometry.

### Abstract of the immunomodulation method

The current therapies for transplant recipient patients are characterized by a very strong immunosuppression, leading to severe problems following the transplantation as infections and tumors. In the present patent application, it is also disclosed an immunomodulation method based on the modulation/MHC II expression reduction in the DCs of the graft, which will migrate to the recipient DLN, duplicating the graft survival period without adding any other medication or immunosuppressing drug. Figure 15 shows that the Hsp70 provides this effect, inducing in the DCs the MARCH-I expression increase, and the subsequent reduction in the MHC II expression, addressed by the IL-10 production via TLR2-ERK-STAT3.

Schematic representation of the signaling route model described on the method of the invention: the modulator, as Hsp70, may bind directly to TLR2 of the DCs, or in an endocytic recipient as described in the state of the art, which may cooperate with TLR2. This binding triggers the ERK activation and the subsequent activation of STAT3, leading to the IL-10 production increase. This IL-10 will induce the MARCH-I expression increase and, thus, reducing the MHC II expression in the DCs membrane.

### Example 2. Process for screening new molecular entities useful for preparing the composition for ex-vivo preservation and/or immunomodulation of organs for transplantation.

Against the surprising experimental results, the inventors investigated if other molecular entities could provide the immunosuppressing effect caused by the MARCH-I, MHC II, CD86 modulation. For this purpose, preservation compositions were initially considered having synthetic analogues of the Hsp70 as, for example, some peptides, and evaluated the binding potential thereof to the MHC II in the animals studied, such that to provide the same immunosuppressant effects. The experiments conceived by the inventors provide a process for screening the new molecular entities useful for preparing the composition for *ex-vivo* preservation and/or immunomodulation of organs for transplantation, the said process comprising:
- a first step of contacting one or more molecular entity(s) with cells having a protein selected from the group consisting of MARCH-I, MHC II, CD86, or combination thereof;
- a second step of evaluating the modulation of the amount of MARCH-I, MHC II and/or CD86 in the cells treated in this way; and
- a third step of selecting molecular entities with major potential of modulation of the MARCH-I, MHC II and/or CD86 amount in the cells treated in this way.

In one embodiment, the said process is wherein the said cells having a protein selected from the group consisting of MARCH-I, MHC II, CD86, or combination thereof, are dendritic cells or cells of the organ to be transplanted itself.

In one embodiment, the said process is wherein the step of evaluating the MARCH-I, MHC II and/or CD86 amount in the cells is done by measuring by Real-time PCR of the mRNA transcribed from genes codifying MARCH-I, MHC II and/or CD86.

In one embodiment, the said process further comprises the step of evaluating *in vitro* the cell viability and/or the presence of cell damage in organs cells after contacting with a composition for organs preservation and/or immunomodulation, as better detailed in the examples 4 and 5.

For performing an investigation on the possibility of using Hsp70 fragments as modulating MARCH-I, MHC II and/or CD86, bioinformatics programs were used for drawing synthetic peptides from the Hsp70 protein sequence being subsequently synthetized by the company JPT Innovative Peptide Solutions (Berlin, Germany). The amino acids sequence of *Mycobacterium tuberculosis* Hsp70 (GI: 61222666) was entered into the IEDB Analysis Resource (http://tools.immuneepitope.org/analyze/html/mhc_II_binding.html), server that previews peptide sequences probability bound to MHC molecules II, providing scores for peptides according to the bonding probability. The smaller the score, higher the peptide probability to bind in the MHCII. Scores smaller than 50 are considered of high affinity, the ones higher than 50 and smaller than 500 are considered of intermediate affinity and the ones higher than 500 and smaller than 5000 are considered of low affinity. Four peptides were then selected of high affinity, two with intermediate affinity and one with low affinity. Subsequently, a sequences line was performed between *M. tuberculosis* Hsp70 and murine Hsp70 (respectively, GI:61222666 and GI:3986773), determining the homology between the peptides. The Table 3 bellow indicates the peptides selected.

**Table 3**

| **Peptide name** | **Amino acids sequence (code of a letter and also three letters)** | **Homology** |
|---|---|---|
| Peptide 1 (SEQ ID No:01) | DKMAMQRLREAAEKAK | Semi-preserved |
| | (Asp-Lys-Met-Ala-Met-Gln-Arg-Leu-Arg-Glu-Ala-Ala-Glu-Lys-Ala-Lys) | |
| Peptide 2 (SEQ ID No:02) | VGGSTRMPAVTDLVK | Semi-preserved |
| | (Val-Gly-Gly-Ser-Thr-Arg-Met-Pro-Ala-Val-Thr-Asp-Leu-Val-Lys) | |
| Peptide 3 (SEQ ID No:03) | KGGVMTRLIERNTTI | Preserved |
| | (Lys-Gly-Gly-Val-Met-Thr-Arg-Leu-Ile-Glu-Arg-Asn-Thr-Thr-Ile) | |
| Peptide 4 (SEQ ID No:04) | PRGIPQIEVTFDIDA | Preserved |
| | (Pro-Arg-Gly-Ile-Pro-Gln-Ile-Glu-Val-Thr-Phe-Asp-Ile-Asp-Ala) | |
| Peptide 5 (SEQ ID No:05) | KYTAPEISARILMKL | Not preserved |
| | (Lys-Tyr-Thr-Ala-Pro-Glu-Ile-Ser-Ala-Arg-Ile-Leu-Met-Lys-Leu) | |
| Peptide 6 (SEQ ID No:06) | ITDAVITTPAYFNDA | Semi-preserved |
| | (Ile-Thr-Asp-Ala-Val-Ile-Thr-Thr-Pro-Ala-Tyr-Phe-Asn-Asp-Ala) | |
| Peptide 7 (SEQ ID No:07) | GSDISAIKSAMEKLG | Not preserved |
| | (Gly-Ser-Asp-Ile-Ser-Ala-Ile-Lys-Ser-Ala-Met-Glu-Lys-Leu-Gly) | |

### The immersion-treatment with a fragment (peptide) of the Hsp70 leads to the cutaneous graft survival increase

According to the results achieved by the inventors, the acute rejection inhibition by the Hsp70 is dependent on Tregs cells CD4+CD25+. In addition, the subcutaneous injection of Hsp70 increased the percentage of the Tregs in the DLN. Recently, it was demonstrated that the MARCH-I expression in the DCs is fundamental for developing thymic Tregs (tTregs). A model explaining all these observations is the fact that the Hsp70 would be introducing the MHC II expression reduction, CD86 and CD80, with the IL-10 production and the MARCH-I increase, would generate induced DCs favoring Tregs generation. Despite the expectation of an increase in the Tregs percentage in the animals' DLN that received grafts treated with Hsp70, it was not possible to note such phenomenon (Figure 7, item C). It is possible that low amounts of antigens are achieving the recipient DLN, which fragments (or peptides) of the Hsp70, being not enough for causing a detectable proliferation of Tregs.

In addition to all these points, there are also evidences that incubated DCs with Hsp70 have in its MHC II peptides of Hsp70, which would be Tregs binders. The treatment of experimental models of autoimmune diseases with peptides of Hsp70 was able to overcome the inflammatory responses by inducing T cells production of IL-10 that were specific to Hsp70.
The inventors also tested other molecular entities as MARCH-I, MHC II and/or CD86 modulators. For this purpose, peptides derived from the Hsp70 protein sequence were synthesized and tested. Four high affinity peptides, two with intermediate affinity and one with low affinity were tested. Subsequently, it was performed the sequences alignment between *M*. *tuberculosis* Hsp70 and mice Hsp70 determining the homology between peptides (Table 4).

**Table 4. Features of the peptides selected from M. tuberculosis Hsp70 sequence.**

| **Peptide name** | **Amino acids sequence** | **Homology** |
|---|---|---|
| Peptide 1 | DKMAMQRLREAAEKAK | Semi-preserved |
| Peptide 2 | VGGSTRMPAVTDLVK | Semi-preserved |
| Peptide 3 | KGGVMTRLIERNTTI | Preserved |
| Peptide 4 | PRGIPQIEVTFDIDA | Preserved |
| Peptide 5 | KYTAPEISARILMKL | Not preserved |
| Peptide 6 | ITDAVITTPAYFNDA | Semi-preserved |
| Peptide 7 | GSDISAIKSAMEKLG | Not preserved |

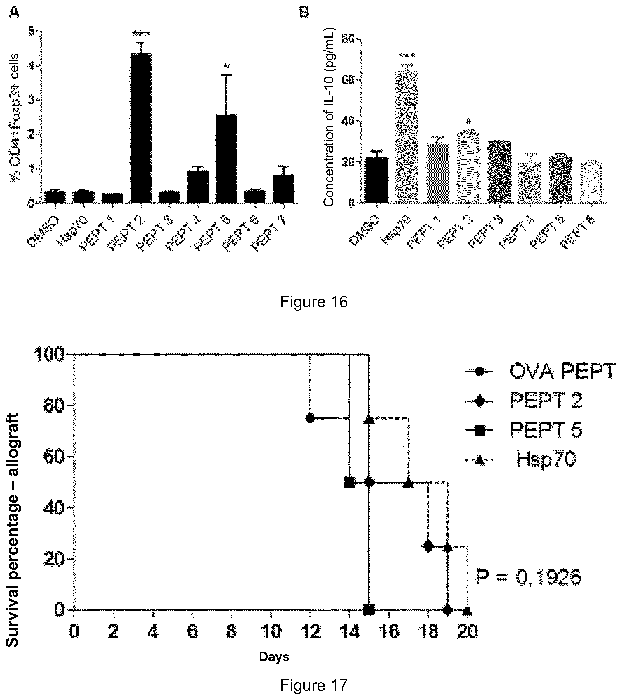

A protocol described on MacLeod and collaborators' job was used, to verify peptides binding selected in the MHC II and the T cells stimulus. For this purpose, BALB/c mice were injected *i.p.* with a solution having 100 µg of the Hsp70 + 7 µg de LPS (served as adjuvant). After nine days, spleen cells (splenocytes) were plates and re-stimulated *in vitro* with DMSO, 60 μg/ml of the Hsp70 or 5 μg/ml of each one of the selected peptides, separately for 24h.After this period, the cultures were analyzed regarding the presence of Tregs by flow cytometry and the supernatants for IL-10 production. We verified, as can be seen in Figure 16 item A, that the peptide 2 was able to generate a quite significant increase in the CD4+Foxp3+ (Tregs) cells percentage when compared with the DMSO.

In addition, the peptide 5 also provided an increase in the Tregs production (Figure 16, item A). However, Hsp70 also did not induce Tregs generation (Figure 16, item A), but in this case because LPS was added in the preparation as adjuvant. If there was no LPS in the system, the Hsp70 would induce these cells in these conditions.

It was also investigated the IL-10 production by splenocytes when stimulated with the selected peptides. For this, the splenocytes *in vitro* were stimulated again resulted from animals treated as described above, with DMSO, Hsp70 or all peptide selected during 24h. After this period, the protein levels of IL-10 were measured in the cultures supernatant. A small increase can be noted in the IL-10 production facing the repeated stimulation with the peptide 2, but not with peptide 5 (Figure 16, item B).

As expected, the treatment with Hsp70 has led to an increase in the IL-10 production by splenocytes (Figure 16, item B). For other peptides, no significant IL-10 production was noted.

Figure 16. Epitopes mapping of T cells from fragments (peptides) of Hsp70. BALB/c mice were injected *i.p.* with a solution containing 100 µg of Hsp70 + 7 µg of LPS. After nine days, the splenocytes were plated and re-stimulated *in vitro* with DMSO (used as negative control, since it was used to dilute peptides), 5 μg/ml of peptide E-α, 60 μg/ml of Hsp70 or 5 μg/ml of each one of the selected peptides, separately for 24h. After this period, (A) the cells were analyzed by flow cytometry for CD4 and CD25 expression or (B) the concentration of IL-10 was determined from the supernatant.

In order to analyze if peptides could inhibit the acute rejection, cutaneous transplantations were performed which B6 WT donors' grafts were treated by immersion with Hsp70, the peptide 2, the peptide 5 or an OVA peptide. After this, these grafts were sutured in BALB/c recipients WT and their rejection were daily monitored.

Surprisingly, peptide 2 presented a strong tendency in lengthen the graft acceptance when compared to OVA peptide (p=) and peptide 5 (p=) (Figure 17). And, as expected, the Hsp70 inhibited the acute rejection when compared to OVA peptide and peptide 5, but not to peptide 2 (Figure 17).

The Table 5 bellow indicated the results between OVAPEPT, PEPT 2 and PET 5, for Hsp70 as well as PEPT2.

**Table 5. Comparison between the results achieved with Hsp70 and PEPT2**

| | **OVA PEPT** | **PEPT 2** | **PEPT 5** |
|---|---|---|---|
| **Hsp70** | * p = 0,0285 | P = 0,4034 | * p = 0,0280 |
| **PEPT 2** | p = 0,0595 | - | p = 0,0598 |

Figure 17 shows the graft treatment with fragment (peptide) of Hsp70 lengthening the cutaneous allograft acceptance in a murine model. Cutaneous grafts of wild (WT) animals B6 (I-A^{b}) were immersed into a solution having 60 μg/ml of Hsp70, 15 μg/ml of peptide 2, peptide 5 or an OVA peptide for 60 minutes at 4°C. After this, the grafts were sutured in the BALB/c (I-Ad) animals' back. The animals were daily monitored. *, p<0.05. n= 4 mice per group.

### Example 3 - Composition for preserving transplantation organs and for rejection inhibition thereof - increase of graft survival.

### The immersion treatment, followed by a topic treatment with Hsp70 increases the cutaneous graft survival

Aiming to lengthen even more the graft acceptance facing the local treatment with Hsp70, we have performed a new strategy in which the recipients that received the treated grafts with Hsp70 or OVA, on day 0.

Subsequently, each animal receives a topic application of 2 drops of Hsp70 or OVA solution until day 7, according to the graft treatment. That is, animals that received grafts treated with Hsp70 receive the local application of a Hsp70 solution and the same for OVA.

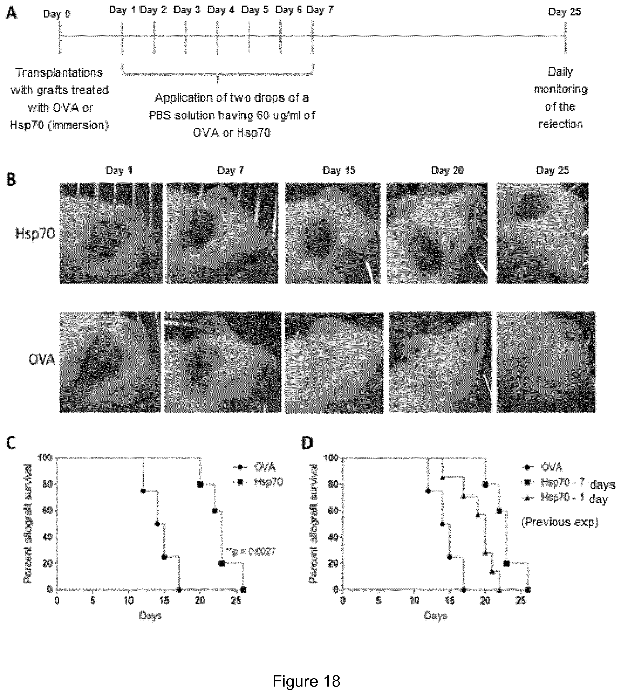

A summary of the strategy used can be found in Figure 18, item A. It can be noted that the graft survival was much bigger in animals treated with Hsp70, when compared to the animals treated with OVA (Figure 18, B and C and Table 6). In addition, this adopted strategy was a bit better than the unique treatment of graft with Hsp70 (Figure 18, item D).

**Table 6: Effect of the graft immersion-treatment with Hsp70 or OVA, followed by 7 days of a topic application of a solution with Hsp70 or OVA in cutaneous allograft murine model. Results achieved when combining the two independent experiments.**

| Treatment | Survival (days) | Survival median | Significance ^{a} |
|---|---|---|---|
| OVA - 7 days | 12, 13, 14, 14, 14, 14, 16 | 14.0 | |
| Hsp70 - 7 days | | | |
| Hsp70 - day | 13, 15, 15, 17, 17, 19, 19, 20 | 17.0 | * |

| | | | |
|---|---|---|---|
| ^{a} Compared to OVA ^{*} P<0,0194 | | | |

**Table 7. Comparison between treatments with Hsp70**

| | OVA | Hsp70 - 1 day |
|---|---|---|
| Hsp70 - 7 days | ** p = 0.0027 | * p = 0.127 |
| Hsp70 - 1 day | * p = 0.0122 | - |

| | | |
|---|---|---|
| *, p<0.05; **, p<0,001. n= 5-7 mice per group. Results obtained when uniting the two independent experiments. | | |

Figure 18 shows the graft treatment with a dose of Hsp70 lengthening the cutaneous allograft acceptance in a murine model. Cutaneous grafts of wild (WT) animals B6 (I-A^{b}) were immersed into a solution having 60 μg/ml of Hsp70 or OVA for 60 minutes at 4°C. After this, the grafts were sutured in BALB/c (I-A^{d}) animals' back. In addition, the animals received a topic application of 2 drops of a solution having 60 μg/ml of Hsp70 or OVA for 7 days in a row. The animals were daily monitored. (A) Schematic representation of the treatment strategy used in the experiment. (B) Photographic record of the clinical progression of the acute rejection in groups Hsp70 and OVA. (C) Graft survival graph of animals that received the treatment described on A. (D) Graft survival graph of animals that received the treatment described on A, compared to animals that received grafts treated with Hsp70 or OVA. Data achieved from previous experiments.

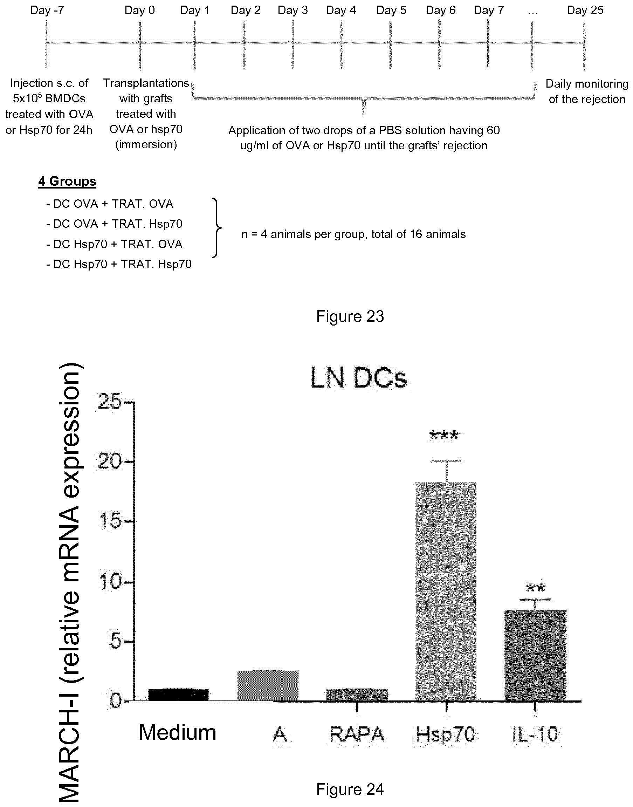

Regarding further immunosuppressant, the treatment with Hsp70 is the one that best induces MARCH-I on dendritic cells. Therefore, the induction of MARCH-I on donor cells has demonstrated a good strategy to length the grafts acceptance. With this, the MARCH-I induction was compared to the one by Hsp70 with two immunosuppressant well used in the clinic for rejection handling and that has been reported to act in dendritic cells modulating the antigen presentation: Cyclosporine A (CsA) and Rapamycin (RAPA). To do so, dendritic cells of lymph nodes were isolated, which were then treated with CsA, RAPA, Hsp70 or IL-10 (positive control) for 24 hours. After this period, the MARCH-I expression increase was analyzed by Real-time PCR. The experimental results as, for example, those indicated on figure 24, show that Hsp70 was the only treatment able to induce MARCH-I on DCs regarding the basal medium. Hsp70 induced MARCH-I on bigger levels when compared to the positive control (IL-10). CsA as well as RAPA were not able of modulating this molecule on DCs when compared to the medium.

### Example 4 - ex vivo organ preservation solutions: evaluation of cell damage

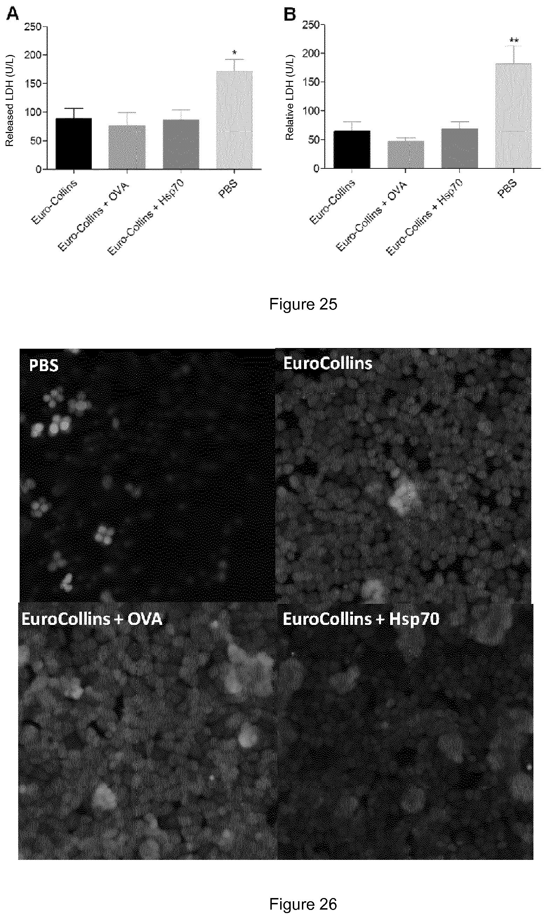

VERO cells were cultivated for 30h on ice, in contact with: 1) an EuroCollins solution; 2) an EuroCollins solution + 30 μg/ml of *M. tuberculosis* Hsp70; 3) an EuroCollins solution + 30 μg/ml of OVA; or 4) PBS 1x (negative control). After this period, the presence of LDH in the supernatant was analyzed (Figure 25 A). The LDH released in comparison to the total LDH present in the cell was evaluated according to what is described in the following (Figure 25 B). In both cases, cells treated with PBS presented a great release of the LDH enzyme, indicating high cell damage. However, when the cells were treated with 1) EuroCollins; 2) EuroCollins+Hsp70; or 3) EuroCollins+OVA, the cell damage was significantly smaller, indicating that the Hsp70 addition to the preservation composition did not result in cell damage or diminishing of detectable cell viability.

### Methodology - VERO cells cultivation

Cells of the renal tissue cell line VERO (ATCC - CCL-81) were cultivated in DMEM (Cultilab) medium supplemented with 10% of bovine fetal serum (FCS) (Cultilab), 1x of essential amino acids (Gibco), 1x vitamins (Gibco) and 55 µM of β-mercaptoethanol at 37°C with 5% of CO₂. 5 x 10⁴ cells per well in a 96 well-plate were plated, being incubated for 24h for cells to adhere. Subsequently, the cultivation medium was removed and the cells submitted to three different treatments: 1) an EuroCollins preservation solution; 2) EuroCollins + 30 μg/ml of *M. tuberculosis* Hsp70; 3) EuroCollins + 30 μg/ml of OVA; or 4) PBS 1x (negative control). Cells were then cultivated for 30h, outside the greenhouse 37°C and in contact with ice, mimicking a *cold storage.*

### Evaluation of cell damage

The presence of cell damage was monitored by detecting the Dehydrogenase Lactic (LDH) in the supernatant of the treated VERO cells and cultivated as defined above. To do so, it was used a kit (Bioclin - K014) for reading in spectrophotometer (GE Healthcare), according to the manufacturer recommendations. Besides monitoring the LDH released, the LDH present in lysates were also monitored, after cell lysis with 1% of Triton in PBS 1x for 15 minutes in stirring. The relative LDH was calculated with the following formula: (LDH released*100)/LDH total.

### Example 5 - ex vivo organ preservation solutions: evaluation of cell viability

The cell viability of treated VERO cells and cultivated for 30h on ice was monitored using LIVE/DEAD® Cell Viability (Life Technologies) kit, according to the manufacturer recommendations. The cells were evaluated in a Confocal Mycroscope (Zeiss).

Besides monitoring LDH, the cells were marked with the Live/Dead kit and analysis by microscopy. In this test, living cells are green and the dead ones red. Cells treated with PBS died much more when compared to other treatments, after 30h of static storage on ice. There was no significant difference in the measured viability proportions, between the EuroCollins group and the EuroCollins+Hsp70 group.

The present data corroborate the invention and provide even more scientific strength to data demonstrating the technical effects of the claimed subject matter. There present data reinforces the conclusion that using MARCH-I, MHC II and/or CD86 is actually useful for compositions of preservation *ex-situ* of transplantation organs. Data show that Hsp70 can be used with an EuroCollins solution not damaging the initial function of preservation, with the additional advantage of providing an immunosuppressant effect - modulating the organ to be transplanted and reducing the rejection.

People skilled in the art will value the knowledge here presented and will be able to reproduce the invention in the embodiments presented and in other forms, covered in the scope of attached claims.

## Claims

1. Use of a modulatory substance of MARCH-I, MHC II and/or CD86 **characterized in that** it is for preparing a composition for *ex-vivo* preservation and immunomodulation of organ cells for transplantation.

2. Composition for *ex-vivo* preservation and immunomodulation of organs for transplantation **characterized by** comprising:
- one or more organ preservation solutions; and
- a substance modulating MARCH-I, MHC II and/or CD86 in the organ cells to be transplanted.

3. Composition according to claim 2 **characterized in that** said organ preservation solutions are chosen from the group consisting of: solution of Winscosin (Viaspan^{®}); Euro-Collins (RENOGRAF^{®}); Histidine-Tryptophan-Ketoglutarate (HTK) solution - Custodiol^{®}; Celsior^{®} Cold Storage Solution; or combinations thereof.

4. Composition according to claim 2 or 3 **characterized in that** the substance or modulating molecular entity of MARCH-I, MHC II and/or CD86 is an Hsp selected from the group consisting of: Hsp70; Hsp60; Hsp40; Hsp90; Hsp110; fragments thereof or combinations of any of those molecular entities.

5. Composition according to claim 4 **characterized in that** said Hsp is the Hsp70 recombinant protein of *M. tuberculosis.*

6. Composition according to claim 4 **characterized in that** the substance or modulating molecular entity of MARCH-I, MHC II and/or CD86 is a peptide selected from the group consisting of peptides identified as: SEQ ID No:01, SEQ ID No:02, SEQ ID No:03, SEQ ID No:04, SEQ ID No:05, SEQ ID No:06; SEQ ID No:07; or combinations thereof.

7. Process for preparing an *ex-vivo* organ cells immunomodulating composition for transplantation **characterized by** comprising a step of mixing one or more organ preservation solutions with at least one modulating molecular entity of the amount of MARCH-I, MHC II and/or CD86.

8. Method for *ex-vivo* immunomodulation of transplantation organs **characterized by** comprising a step of *ex-vivo* contacting the organ cells with one or more substance(s) modulating MARCH-I, MHC II and/or CD86.

9. Method according to claim 8 **characterized in that** said *ex-vivo* organ is selected from the group consisting of skin, heart and/or kidney.

10. Process for screening new molecular entities useful for preparing the composition for *ex-vivo* preservation and/or immunomodulation of transplantation organs **characterized by** comprising:
- a first step of contacting one or more molecular entity(ies) with cells having a protein selected from the group consisting of MARCH-I, MHC II, CD86, or combinations thereof;
- a second step of evaluating the modulation of the amount of MARCH-I, MHC II and/or CD86 in the cells treated in this way; and
- a third step of selecting the molecular entities with major potential of the modulation of the amount of MARCH-I, MHC II and/or CD86 in the cells treated in this way.

11. Process according to claim 10 **characterized in that** said cells having a protein selected from the group consisting of MARCH-I, MHC II, CD86, or combinations thereof, are dendritic cells or cells of the organ to be transplanted itself.

12. Process according to claim 10 or 11 **characterized in that** the step of evaluating the amount of MARCH-I, MHC II and/or CD86 in the cells is done by measuring through PCR in real time the mRNA transcribed from genes codifying MARCH-I, MHC II and/or CD86.

13. Process according to claim 10, 11 or 12 **characterized by** further comprising the step of *in vitro* evaluating the cell viability and/or the presence of cell damage in the organ cells *ex-vivo* after contacting said modulating molecular entities of the amount of MARCH-I, MHC II and/or CD86.
